(19) Europäisches Patentamt
European Patent Office
Office européen des brevets

(11) **EP 3 603 900 B1**

(12) **EUROPEAN PATENT SPECIFICATION**

(45) Date of publication and mention
of the grant of the patent:
**13.07.2022 Bulletin 2022/28**

(21) Application number: **18777117.5**

(22) Date of filing: **11.02.2018**

(51) International Patent Classification (IPC):
**B25J 9/06** (2006.01)   **A61B 34/30** (2016.01)
**A61B 17/00** (2006.01)   **A61B 1/005** (2006.01)

(52) Cooperative Patent Classification (CPC):
**A61B 1/0055; A61B 17/00234; A61B 34/71;
B25J 9/06; B25J 17/00;** A61B 1/0057;
A61B 2017/00314; A61B 2017/0069;
A61B 2034/301; A61B 2034/306

(86) International application number:
**PCT/CN2018/076315**

(87) International publication number:
**WO 2018/177040 (04.10.2018 Gazette 2018/40)**

(54) **SNAKE-LIKE JOINT FOR SURGICAL ROBOT, SURGICAL INSTRUMENT, AND ENDOSCOPE**

SCHLANGENFÖRMIGES GELENK FÜR EINEN CHIRURGISCHEN ROBOTER, CHIRURGISCHES INSTRUMENT UND ENDOSKOP

ARTICULATION DE TYPE SERPENT POUR ROBOT CHIRURGICAL, INSTRUMENT CHIRURGICAL ET ENDOSCOPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2017   CN 201710203999**

(43) Date of publication of application:
**05.02.2020   Bulletin 2020/06**

(73) Proprietor: **Shanghai Microport Medbot (Group)
Co., Ltd.
Shanghai 201203 (CN)**

(72) Inventors:
• **HE, Yuyuan
Shanghai 201203 (CN)**
• **HE, Chao
Shanghai 201203 (CN)**

• **WANG, Changchun
Shanghai 201203 (CN)**
• **LI, Tao
Shanghai 201203 (CN)**

(74) Representative: **Patentship
Patentanwaltsgesellschaft mbH
Elsenheimerstraße 65
80687 München (DE)**

(56) References cited:
CN-A- 101 106 935    CN-A- 101 940 462
CN-A- 102 497 801    CN-A- 106 963 494
CN-U- 204 192 566    CN-U- 204 192 566
CN-U- 205 612 443    US-A1- 2008 132 761
US-A1- 2016 066 937    US-B2- 9 387 585

## Description

TECHNICAL FIELD

The present application relates to the technical field of medical instruments, in particular, to a snake-like joint for a surgical robot, a surgical instrument and an endoscope.

BACKGROUND

With the rapid development in the field of robots, various distinctive robots have emerged constantly, among which, the researches on bionic robots such as the snake-like robots is one of the hottest. Different from the other robots, the snake-like robot can move similar to a snake in nature, being able to achieve planar torsion and spatial torsion in order to avoid obstacles during movements. So the snake-like robot is used to accomplish tasks that cannot be accomplished by humans or other machines.

Based on this feature, snake-like robots for medical operations have also appeared, especially for the minimally invasive surgery. Due to the small wound in the minimally invasive surgery, the surgical instruments having snake-like joints of the snake-like robots can avoid collision to the other organs to reduce the risk of organ's damage during the surgery, such that a better therapeutic effect is achieved. This advantage makes the surgical instruments having snake-like joints well applied to the medical field.

However, the snake-like joints for the medical instruments used currently have the disadvantages of a complex structure, a numerous of components, and a complicated assembly.

The Chinese Patent Application Publication, CN 204 192 566 U, discloses a unit joint comprising a barrel and supporting lugs, first positioning parts being provided on the wall of the barrel, second positioning parts being provided on the supporting lugs. The barrel and/or the supporting lugs are made of plastic materials, thereby having the plastic deformation capacity. In the installation process, the plastic portions on two unit joints can be squeezed by hand, so that the second locating parts or the first locating parts on the supporting lugs are connected with the first locating parts or the second locating parts on the other unit joint. After squeezing force is removed, deformation of the plastic portions is eliminated, and connection between the two adjacent joints can be achieved.

The US Patent, US 9,387,585 B2, discloses a two-far end supported actuator module for a snake robot using an inner wire which includes: a module housing having a driving means and a reducer mounted therein, and having a plurality of accommodation portions on an outer surface thereof; an upper cover and a lower cover detachably installed at an upper part and a lower part of the module housing, respectively, wherein the upper cover has a rotation shaft passing hole for passing a rotation shaft therethrough, and the lower cover has a wire passing hole for passing a wire therethrough; a first connection bracket having one side coupled to one accommodation portion of the module housing, and another side coupled to a lower cover of another driving module, and configured to guide the wire thereinto; and a second connection bracket coupled to another accommodation portion of the module housing on the opposite side of the first connection bracket, and another side to which a rotation shaft is inserted to be supported.

A snake-like joint proposed in US Patent Application Publication No. 2016/0066937A1 is shown in FIG. 17. A single joint 110 includes a first joint portion 202 and a second joint portion 232 capable of swinging relative to the first joint portion 202, which are connected via short link mechanisms 226, 228. Taking the short link mechanism 226 as an example, the short link mechanism 226 includes a first bearing 227 coupled in a bearing hole 254 of the second joint portion 232, and a second bearing 225 coupled in a groove 210 of the first joint portion 202, the first bearing 227 forming the rotary shaft of the second joint portion 232, the second bearing 225 forming the rotary shaft of the second joint portion 202. The snake-like joint can realize movements of spatial torsion through different arrangements, but has the disadvantages of a low motion precision and a complicated structure in the process of bending or torsion.

As shown in FIG. 18, the Chinese patent application CN105078398A discloses a unit section of a snake-bone-shaped tube 100, including a barrel 110 and a lug 120, the barrel 110 having a connecting hole 113 formed therein, the lug 120 having a pin 121 disposed thereon. A snake-bone-shaped tube structure can be formed when a plurality of unit sections of the snake-bone-shaped tube 100 are successively hinged end to end via the connecting holes 113 and the pins 121. However, the snake-bone-shape tube structure has only one degree of freedom and cannot achieve complicated spatial torsion, thus limiting its application.

SUMMARY

The invention is as defined by independent claim 1. Dependent claims disclose exemplary embodiments.

It is an object of some exemplary embodiments to provide a snake-like joint for a surgical robot to improve the motion accuracy or reduce the complexity of the structure of the snake-like joint. The object of other exemplary embodiments is to provide a surgical instrument having a snake-like joint and an endoscope having a snake-like joint.

Based on the foregoing objectives, embodiments according to the invention provide a snake-like joint for a surgical robot, having at least one degree of freedom, and comprising at least one joint connector pair and a flexible structure, wherein,

each joint connector pair comprises a first joint connector at a lower end and a second joint connector at an upper end; wherein the first joint connector comprises a first base as well as a first support structure and a second support structure disposed on a first surface of the first base, the first support structure having a first protrusion formed thereon, the second support structure having a first connecting hole formed therein ; wherein the second joint connector comprises a second base as well as a third support structure and a fourth support structure disposed on a second surface of the second base; the third support structure having a second protrusion formed thereon, the fourth support structure having a second connecting hole formed therein; when the first joint connector and the second joint connector are engaged with each other, the first surface and the second surface are disposed opposite to each other, the first protrusion being configured to locate in the second connecting hole and rotate relative to the second connecting hole, the second protrusion being configured to locate in the first connecting hole and rotate relative to the first connecting hole; the flexible structure controls the joint connector pair to swing around a joint axis.

Optionally, in the snake-like joint for a surgical robot, the first protrusion has a revolving body structure formed by a straight line or a curve around a first axis, and the first connecting hole has a revolving surface structure formed by a straight line or a curve around a second axis, each of the first axis and the second axis being vertically intersected with an axis of the first base; wherein the second protrusion has a revolving body structure formed by a straight line or a curve around a third axis, and the second connecting hole has a revolving surface structure formed by a straight line or a curve around a fourth axis; and when the first joint connector and the second joint connector are engaged with each other, the joint axis, the first axis, the second axis, the third axis, and the fourth axis are collinear.

Optionally, in the snake-like joint for a surgical robot, each of the first protrusion and the second protrusion has a structure of a cylinder, a circular truncated cone, or a conical cone; and each of the first connecting hole and the second connecting hole has a structure of a cylindrical surface structures, a circular truncated cone surface, or a circular conical surface.

According to a first example of the invention, in the snake-like joint for a surgical robot, the first protrusion is disposed on a first side of the first support structure, the first side being a side of the first support structure away from the axis of the first base;

a side of the first support structure close to the axis of the first base is a fifth side; the second protrusion is disposed on a second side of the third support structure, the second side being a side of the third support structure away from an axis of the second base; a side of the third support structure close to the axis of the second base is a sixth side; a side of the second support structure close to the axis of the first base is a third side; a side of the fourth support structure close to the axis of the second base is a fourth side; a distance l1 between the first side and the third side and a distance l2 between the second side and the fourth side have the following relationship:

$$l1-lp2<l2<l1+lp1,$$

or,

$$l2-lp1<l1<l2+lp2,$$

wherein lp1 is an axial length of the first protrusion, and lp2 is an axial length of the second protrusion; and, a sum of a distance between the first side and the fifth side and the axial length of the first protrusion is smaller than a distance between the fourth side and the sixth side; a sum of the axial length of the second protrusion and a distance between the second side and the sixth side is smaller than a distance between the third side and the fifth side.

Optionally, in the snake-like joint for a surgical robot, a sum of the axial length of the first protrusion and a distance from the first side of the first support structure to a seventh side of the second support structure is smaller than or equal to a diameter of the first base, wherein a side of the second support structure away from the axis of the first base is the seventh side;

a sum of the axial length of the second protrusion and a distance from the second side of the third support structure to an eighth side of the fourth support structure is smaller than or equal to a diameter of the second base, wherein a side of the fourth support structure away from the axis of the second base is the eighth side; in the joint connector pair, a distance from an outer end face of the first protrusion to an outer end face of the second protrusion is smaller than or equal to the diameter of the first base, and in the joint connector pair, the distance from the outer end face of the first protrusion to the outer end face of the second protrusion is also smaller than or equal to the diameter of the second base.

According to a second example of the invention, in the snake-like joint for a surgical robot, a side of the second support structure close to an axis of the first base is a third side;

a side of the fourth support structure close to an axis of the second base is a fourth side;
the first protrusion is located on a fifth side of the first support structure, the fifth side being a side of the first support structure close to the axis of the first base;
the second protrusion is located on a sixth side of the third support structure, the sixth side being a side of the third support structure close to the axis of the second base;
a side of the second support structure away from the axis of the first base is a seventh side;
a side of the fourth support structure away from the axis of the second base is an eighth side;
a distance l3 between the fifth side and the seventh side and a distance l4 between the sixth side and the eighth side have the following relationship:

$$l3-lp1<l4<l3+lp2,$$

or,

$$l4-lp2<l3<l4+lp1,$$

wherein lp1 is an axial length of the first protrusion, and lp2 is an axial length of the second protrusion; and,
a distance between the fourth side and the eighth side is smaller than a difference of a distance between the third side and the fifth side and the axial length of the first protrusion;
a distance between the third side and the seventh side is smaller than a difference of a distance between the fourth side and the sixth side and the axial length of the second protrusion.

Optionally, in the snake-like joint for a surgical robot, a distance from a first side of the first support structure to the seventh side of the second support structure is smaller than or equal to a diameter of the first base, wherein the first side is a side of the first support structure away from the axis of the first base;

a distance from the second side of the third support structure to the eighth side of the fourth support structure is smaller than or equal to a diameter of the second base, wherein the second side is a side of the third support structure away from the axis of the second base;
in the joint connector pair, a distance from the first side to the second side is smaller than or equal to the diameter of the first base, and in the joint connector pair, the distance from the first side to the second side is also smaller than or equal to the diameter of the second base.

Optionally, in the snake-like joint for a surgical robot, a thickness of the first support structure, a thickness of the second support structure, and the axial length of the first protrusion are equal; and a thickness of the third support structure, a thickness of the fourth support structure, and the axial length of the second protrusion are equal.

Optionally, in the snake-like joint for a surgical robot, the first surface comprises a first partial surface and a second partial surface intersected with the first partial surface, each of the first support structure and the second support structure being disposed on the first partial surface, and wherein the second surface comprises a third partial surface and a fourth partial surface intersected with the third partial surface, each of the third support structure and the fourth support structure being disposed on the third partial surface.

Optionally, in the snake-like joint for a surgical robot, the second partial surface is a plane, and the joint axis passes through a plane where the second partial surface is located;
the fourth partial surface is a plane, and the joint axis passes through a plane where the fourth partial surface is located.

Optionally, in the snake-like joint for a surgical robot, an angle between the second partial surface and the first partial surface is 0°-45°, and an angle between the fourth partial surface and the third partial surface is 0°-45°.

Optionally, in the snake-like joint for a surgical robot, the second partial surface and the fourth partial surface are both curved surfaces configured such that when the joint connector pair swings to a condition that the second partial surface is in contact with the fourth partial surface on a same side, a contact portion is formed between the second partial surface and the fourth partial surface, and a common tangent plane is present between the curved surfaces at the contact portion with the first axis passing through the common tangent plane.

Optionally, in the snake-like joint for a surgical robot, an angle between the common tangent plane and the first partial surface is greater than 0° and smaller than or equal to 45°, and an angle between the common tangent plane and the third partial surface is greater than 0° and smaller than or equal to 45°.

Optionally, in the snake-like joint for a surgical robot, a swing angle between the first joint connector and the second joint connector in each joint connector pair is greater than 0° and smaller than or equal to 90°.

Optionally, in the snake-like joint for a surgical robot, first axial through holes are disposed in a circumference of the first base, and the first axial through holes are provided for the flexible structure to extend through; wherein second axial through holes are formed in a circumference of the second base, and the second axial through holes are provided for the flexible structure to extend through.

Optionally, in the snake-like joint for a surgical robot, a number of the first axial through holes and a number of the second axial through holes are plural, and positions of at least two of the first axial through holes correspond to positions of at least two of the second axial through holes.

Optionally, in the snake-like joint for a surgical robot, in each joint connector pair, the number of the first axial through holes is the same as the number of the second axial through holes, and positions of the first axial through holes have a one-to-one correspondence to positions of the second axial through holes.

Optionally, in the snake-like joint for a surgical robot, there are at least 2n flexible structures when the snake-like joint for the surgical robot has n degree(s) of freedom, wherein n is a natural number greater than or equal to 1.

Optionally, when the snake-like joint for a surgical robot comprises a plurality of joint connector pairs, adjacent joint connector pairs are detachably or fixedly connected.

Optionally, in the snake-like joint for a surgical robot, a first fastening mechanism is disposed on a third surface of the first base, the third surface being opposite to the first surface; and a second fastening mechanism is disposed on a fourth surface of the second base, the fourth surface being opposite to the second surface; wherein the second fastening mechanism of one joint connector pair is lockable with a first fastening mechanism of an adjacent joint connector pairs, so that the second joint connector of the one joint connector pair is detachably connected to the first joint connector of the adjacent joint connector pair.

Optionally, in the snake-like joint for a surgical robot, the first fastening mechanism comprises a plurality of first bosses and a plurality of first recesses, and the first bosses and the first recesses are arranged at intervals; wherein the second fastening mechanism comprises a plurality of second bosses and a plurality of second recesses, and the second bosses and the second recesses are arranged at intervals; wherein the first bosses correspond to the second recesses in shape, number and position, and the second bosses correspond to the first recesses in shape, number and position.

Optionally, in the snake-like joint for a surgical robot, in adjacent joint connector pairs, a second joint connector of one connector pair at a proximal end is integrally formed with a first joint connector of the other joint connector pair at a distal end.

Optionally, in the snake-like joint for a surgical robot, a circumferential offset angle of adjacent joint connector pairs is greater than or equal to 0° and smaller than or equal to 180°.

Another exemplary embodiment provides a surgical instrument, comprising: an instrument terminal, a snake-like joint for a surgical robot as stated above, a tubular member, a flexible member, and a controller, wherein the instrument terminal, the snake-like joint for a surgical robot, the tubular member, and the controller are sequentially connected from distal end to proximal end, one end of the flexible member being connected to the controller with the other end of the flexible member being connected to the instrument terminal after passing through the tubular member, a proximal end of a flexible structure of the snake-like joint for a surgical robot being connected to the controller after passing through the tubular member, the controller controlling movements of the instrument terminal through the flexible member, and controlling swings of the snake-like joint for a surgical robot through the flexible structure of the snake-like joint for a surgical robot.

Still another embodiment provides an endoscope, comprising: an imaging system, a snake-like joint for a surgical robot as stated above, a tubular member, and a controller, wherein the imaging system, the snake-like joint for a surgical robot, the tubular member, and the controller are sequentially connected from distal end to proximal end; a proximal end of a flexible structure of the snake-like joint for a surgical robot being connected to the controller after passing through the tubular member, the controller controlling swings of the snake-like joint for a surgical robot through the flexible structure of the snake-like joint for a surgical robot, so as to adjust a posture of the imaging system.

The snake-like joint for a surgical robot provided in some exemplary embodiments achieves the planar torsion or spatial torsion through swings of the joint connector pair, enabling to reduce the complexity of structure thereof compared to the snake-like joint in the prior art. The surgical instrument and the endoscope provided in other exemplary embodiments include the snake-like joint for a surgical robot, thereby enabling the instrument terminal or the imaging system to reach a desired position and posture.

BRIEF DESCRIPTION OF THE DRAWINGS

FIG. 1 is a perspective view showing a separated state of one joint connector pair according to a snake-like joint for a surgical robot of one exemplary embodiment;
FIG. 2 is a schematic front view of the joint connector pair shown in FIG. 1;
FIG. 3 is a schematic front view showing a connected state of the joint connector pair shown in FIG. 2;
FIG. 4 is a schematic top view of a first base /second base according to a snake-like joint for a surgical robot of one exemplary embodiment;
FIG. 5 is another schematic top view of the first base /second base according to a snake-like joint for a surgical robot of one exemplary embodiment;

FIG. 6 is a perspective view showing a separated state of a second joint connector in one joint connector pair and the adjacent first joint connector in adjacent joint connector pair according to a snake-like joint for a surgical robot of one exemplary embodiment;

FIG. 7 is a schematic front view of the second joint connector in one joint connector pair and the adjacent first joint connector in adjacent joint connector pair shown in FIG. 6;

FIG. 8 is a schematic front view showing a connected state of the second joint connector in one joint connector pair and the adjacent first joint connector in adjacent joint connector pair shown in FIG. 7;

FIG. 9 is a perspective view showing a separated state of a second joint connector in one joint connector pair and the adjacent first joint connector in adjacent joint connector pair according to a snake-like joint for a surgical robot of one exemplary embodiment;

FIG. 10 is a schematic front view of the second joint connector in one joint connector pair and the adjacent first joint connector in adjacent joint connector pair shown in FIG. 9;

FIG. 11 is a schematic front view showing a connected state of the second joint connector in one joint connector pair and the adjacent first joint connector in adjacent joint connector pair shown in FIG. 10;

FIG. 12 is a perspective view showing a separated state of a plurality of joint connector pairs in the snake-like joint for a surgical robot according to one exemplary embodiment;

FIG. 13 is a perspective view showing a curved state of a plurality of joint connector pairs shown in FIG. 12;

FIG. 14 is a schematic front view showing a curved state of a plurality of joint connector pairs shown in FIG. 13;

FIG. 15 is a schematic structural view showing a natural state of a surgical instrument according to one exemplary embodiment;

FIG. 16 is a schematic structural view showing a rotating state of the surgical instrument according to one exemplary embodiment;

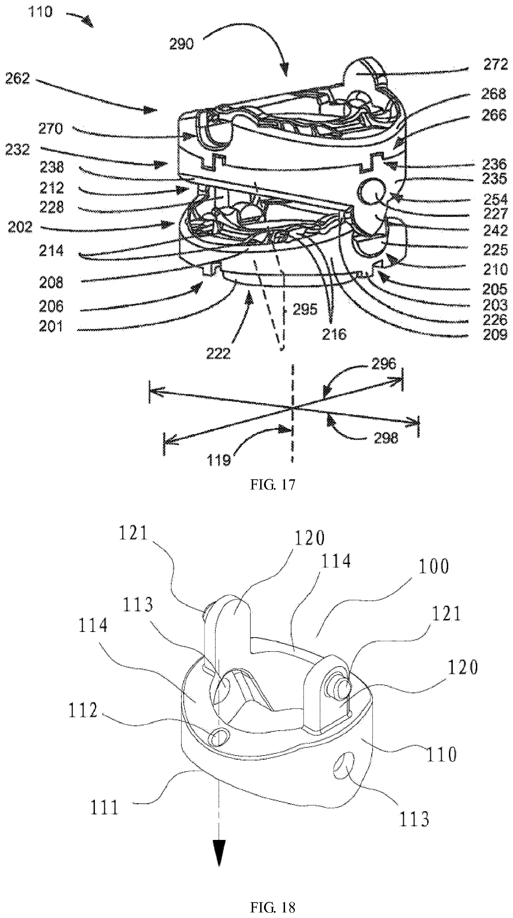

FIG. 17 is a schematic view of a snake-like joint in the prior art;

FIG. 18 is a schematic view of a unit section of a snake-bone-shaped tube in the prior art.

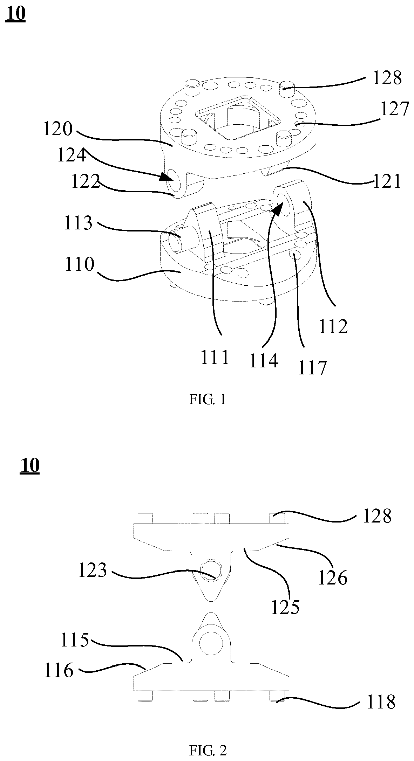

In FIG. 1-FIG. 16: joint connector pairs-10, 10a, 10b, 10c, 10d; first joint connectors-11, 11a, 11b, 11c, 11d; second joint connectors-12, 12a, 12b, 12c, 12d; first support structure-111;second support structure-112;third support structure-121; fourth support structure-122; first protrusion-113; second protrusion-123;first connecting hole-114;second connecting hole-124; first partial surface-115;second partial surface 116; third partial surface-125; fourth partial surface 126; first axial through hole-117; second axial through hole-127; first fastening mechanism-118; second fastening mechanism-128; snake-like joint for a surgical robot-1; instrument terminal-2.

## DETAILED DESCRIPTION

The snake-like joint for a surgical robot, the surgical instrument and the endoscope proposed in the present application will be further described in detail below with reference to the accompanying drawings and specific embodiments. Advantages and features of the present application will be more apparent from the description and appended claims below. It should be noted that the accompanying drawings are presented in a very simplified form and not necessarily presented to scale, with the only intention to facilitate convenience and clarity in explaining the object of the present application. In particular, the drawings show different emphases, and often adopt different proportions.

In the present application, "proximal end", "distal end" or "upper end", "lower end" are opposing orientations, positions, and directions of elements or actions facing each other from the perspective of a doctor operating the medical instrument. Although the "proximal end" and "distal end", or "upper end" and "lower end" are not restrictive, the "proximal end" and "lower end" generally refer to an end of the medical device close to the operator during the normal operation, and the "distal end" and "upper end" generally refer to an end away from the operator.

The main idea of some exemplary embodiments is to provide a snake-like joint for a surgical robot, comprising at least one joint connector pair and a flexible structure, wherein each joint connector pair comprises a first joint connector at the lower end of the joint connector pair and a second joint connector at the upper end of the joint connector pair; wherein the first joint connector comprises a first base as well as a first support structure and a second support structure disposed on a first surface of the first base, the first support structure having a first protrusion formed thereon, the second support structure having a first connecting hole formed therein; wherein the second joint connector comprises a second base as well as a third support structure and a fourth support structure disposed on a second surface of the second base, the third support structure having a second protrusion formed thereon, the fourth support structure having a second connecting hole formed therein; wherein the first surface and the second surface are disposed opposite to each other, the first protrusion being configured to locate in the second connecting hole and rotate relative to the second connecting hole, the second protrusion being configured to locate in the first connecting hole and rotate relative to the first connecting hole; and wherein the flexible structure controls the joint connector pair to swing around a joint axis. By means of the swing of the joint connector pair, the snake-like joint for a surgical robot achieves the planar torsion or spatial torsion, reduces its complexity of structure compared with the snake-like joint in the prior art. Further, a surgical instrument and an endoscope which comprise the snake-like joint for a surgical robot are provided in other exemplary embodiments, so that an instrument terminal of the surgical instrument or an imaging system is able to reach a desired position and posture.

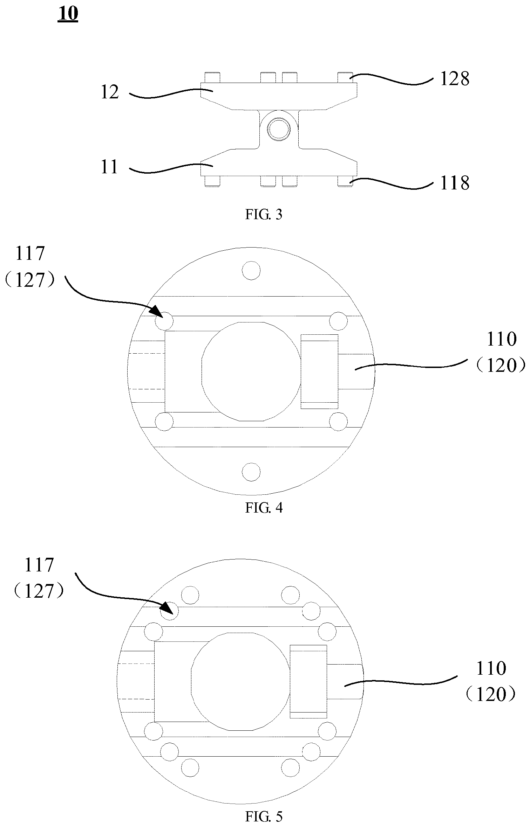

Specifically, referring to FIG. 1-FIG. 3, FIG. 1 is a perspective view showing a separated state of one joint connector pair according to a snake-like joint for a surgical robot of one exemplary embodiment; FIG. 2 is a schematic front view of the joint connector pair shown in FIG. 1; and FIG. 3 is a schematic front view showing a connected state of the joint connector pair shown in FIG. 2. As shown in FIG. 1-FIG. 3, each joint connector pair 10 comprises a first joint connector 11 at the lower end of the joint connector pair 10 and a second joint connector 12 at the upper end of the joint connector pair 10. The first joint connector 11 comprises a first base 110 as well as the first support structure 111 and the second support structure 112 disposed on the first surface of the first base 110, the first support structure 111 having the first protrusion 113 formed thereon, the second support structure 112 having the first connecting hole 114 formed therein. The second joint connector 12 comprises a second base 120 as well as the third support structure 121 and a fourth support structure 122 disposed on the second surface of the second base 120, the third support structure 121 having the second protrusion 123 formed thereon, the fourth support structure 122 having the second connecting hole 124 formed therein. The first surface and the second surface are disposed opposite to each other, the first protrusion 113 being configured to locate in the second connecting hole 124 and rotate relative to the second connecting hole, the second protrusion 123 being configured to locate in the first connecting hole 114 and rotate relative to the first connecting hole.

In one exemplary embodiment, the first protrusion 113 has a revolving body structure formed by a straight line or a curve around a first axis, and the first connecting hole 114 has a revolving surface structure formed by a corresponding straight line or a curve around a second axis, each of the first axis and the second axis being vertically intersected with the axis of the first base 110. The second protrusion 123 has a revolving body structure formed by a straight line or a curve around a third axis, and the second connecting hole 124 has a revolving surface structure formed by a corresponding straight line or a curve around a fourth axis, the third axis and the fourth axis being vertically intersected with the axis of the second base 120. Further, the first axis and the second axis are collinear, and the third axis and the fourth axis are collinear. In the connected state of the joint connector pair shown in FIG. 3, the first axis, the second axis, the third axis, and the fourth axis in the joint connector pair 10 are collinear. It is obvious that the first axis, the second axis, the third axis, and the fourth axis herein are also collinear with the joint axis of the joint connector pair 10. Therefore, the first protrusion 113 is well fitted into the second connecting hole 124, and the second protrusion 123 is well fitted into the first connecting hole 114, so as to ensure the stability and reliability of the first joint connector 11 and the second joint connector 12 during the swinging process. More specifically, the first protrusion 113 and the second protrusion 123 may have a structure of a cylinder, a circular truncated cone, a circular cone, and so on. The first connecting hole 114 and the second connecting hole 124 may have a structure of a cylinder surface, a circular truncated cone surface, a circular conical surface, and so on.

Still referring to FIG. 1-FIG .3, in one exemplary embodiment, the first protrusion 113 is disposed on the first side of the first support structure 111, the first side being a side of the first support structure 111 away from the axis of the first base 110. The side of the first support structure 111 close to the axis of the first base 110 is the fifth side. The second protrusion 123 is located on the second side of the third support structure 121, the second side being a side of the third support structure 121 away from the axis of the second base 120. The side of the third support structure 121 close to the axis of the second base 120 is the sixth side. The side of the second support structure 112 close to the axis of the first base 110 is the third side. The side of the fourth support structure 122 close to the axis of the second base 120 is the fourth side. The side of the second support structure 112 away from the axis of the first base 110 is the seventh side. The side of the fourth support structure 122 away from the axis of the second base 120 is the eighth side. Preferably, the first to eighth sides are parallel to each other. The distance 11 between the first side and the third side and the distance 12 between the second side and the fourth side have the following relationship: $l1-lp2<l2<l1+lp1$, or, $l2-lp1<l1<l2+lp2$, wherein lp1 is the axial length of the first protrusion, and lp2 is the axial length of the second protrusion. Meanwhile, the sum of the axial length of the first protrusion and the distance between the first side and the fifth side is smaller than the distance between the fourth side and the sixth side. The sum of the axial length of the second protrusion and the distance between the second side and the sixth side is smaller than the distance between the third side and the fifth side. The distance from one side to the other side herein is measured by the length of the joint axis between two sides. The axis length of the first protrusion or the second protrusion is also measured by the length of the joint axis between the end face of the corresponding protrusion and the side adjacent to the corresponding protrusion. It is also applicable to the following embodiments. In this case, the connection between the first joint connector 11 and the second joint connector 12 is able to be achieved, i.e., the first protrusion 113 being configured to dispose in the second connecting hole 124, and the second protrusion 123 being configured to dispose in the first connecting hole 114.

Preferably, the sum of the axial length of the first protrusion 113 and the distance from the first side of the first support structure 111 to the seventh side of the second support structure 112 is smaller than or equal to the diameter of the first base 110. The sum of the axial length of the second protrusion 123 and the distance from the second side of the third support structure 121 to the eighth side of the fourth support structure 122 is smaller than or equal to the diameter of the second base 120. In a joint connector pair, the distance from the outer end face of the first protrusion 113 to the outer end face of the second protrusion 123 is smaller than or equal to the diameter of the first base 110, as well as being smaller than or equal to the diameter of the second base 120 after assembly. That is, the first support structure 111, the second support structure 112, and the first protrusion 113 are all located within the projection of the first base 110, and the third support structure 121, the fourth support structure 122, and the second protrusion 123 are all located within the projection of the second base 120, thereby enabling to improve the safety and reliability of the snake-like joint for a surgical robot are higher, and avoid the first protrusion 113 or the second protrusion 123 from scratching human tissues or the like in use.

In some exemplary embodiments, the side of the second support structure 112 close to the axis of the first base 110 is the third side. The side of the fourth support structure 122 close to the axis of the second base 120 is the fourth side. The first protrusion 113 may also be located on the fifth side of the first support structure 111, the fifth side being the side of the first support structure 111 close to the axis of the first base 110. The second protrusion 123 is located on the sixth side of the third support structure 121, the sixth side being the side of the third support structure 121 close to the axis of the second base 120. The side of the second support structure 112 away from the axis of the first base 110 is the seventh side. The side of the fourth support structure 122 away from the axis of the second base 120 is the eighth side. The side of the first support structure 111 away from the axis of the first base 110 is the first side. The side of the third support structure 121 away from the axis of the second base 120 is the second side. Preferably, the first to eighth sides are parallel to each other. The distance 13 between the fifth side and the seventh side and the distance 14 between the sixth side and the eighth side have the following relationship: l3-lp1<l4<l3+lp2, wherein lp2 is the axial length of the second protrusion, or 14-lp2<l3<l4+lp1, wherein lp1 is the axial length of the first protrusion. Meanwhile, the distance between the fourth side and the eighth side is smaller than the difference of the distance between the third side and the fifth side and the axial length of the first protrusion. The distance between the third side and the seventh side is smaller than the difference of the distance between the fourth side and the sixth side and the axial length of the second protrusion.

Similarly, the distance from the first side of the first support structure 111 to the seventh side of the second support structure 112 is smaller than or equal to the diameter of the first base 110. The distance from the second side of the third support structure 121 to the eighth side of the fourth support structure 122 is smaller than or equal to the diameter of the second base 120. In a joint connector pair, the distance from the first side to the second side is smaller than or equal to the diameter of the first base 110, as well as being smaller than or equal to the diameter of the second base 120 after assembly. That is, the first support structure 111, the second support structure 112, and the first protrusion 113 are all located within the projection of the first base 110, and the third support structure 121, the fourth support structure 122, and the second protrusion 123 are all located within the projection of the second base 120, thereby enabling to improve the safety and reliability of the snake-like joint for a surgical robot and avoid the first side and the second side from scratching human tissues or the like in use.

More preferably, the thickness of the first support structure 111 (i.e., the distance between the first side and the fifth side), the thickness of the second support structure 112 (i.e., the distance between the third side and the seventh side), and the axial length of the first protrusion 113 are equal. The thickness of the third support structure 121 (i.e., the distance between the second side and the sixth side), the thickness of the fourth support structure 122 (i.e., the distance between the fourth side and the eighth side), and the axial length of the second protrusion 123 are equal. So it is provided a better stability of the joint connector pair 10 and an easier replacement of the joint connector.

Still referring to FIG. 1-FIG. 3, in one exemplary embodiment, the first surface includes a first partial surface 115 and two second partial surfaces 116 intersected with the first partial surface 115 and disposed at both sides of the joint axis. Each of the first support structure 111 and the second support structure 112 is disposed on, preferably perpendicular to the first partial surface 115. The two second partial surfaces 116 are preferably arranged symmetrically about the joint axis. The second surface includes a third partial surface 125 and two fourth partial surfaces 126 intersected with the third partial surface 125 and disposed at both sides of the joint axis. Each of the third support structure 121 and the fourth support structure 122 is disposed on, preferably perpendicular to the third partial surface 125. The two fourth partial surfaces 126 are preferably arranged symmetrically about the joint axis. Preferably, in the connected state of the joint connector pair shown in FIG. 3, when one of the second partial surfaces 116 is in contact with one of the fourth partial surfaces 126 at a same side, the second partial surface 116 and the fourth partial surface 126 are in a same plane, and the first axis and the third axis are also in this plane. Further, an angle between the second partial surface 116 and the first partial surface 115 is 0°-45°, and an angle between the fourth partial surface 126 and the third partial surface 125 is 0°-45°. In addition, the second partial surface 116 may be a first curved surface, and the fourth partial surface 126 may be a second curved surface. Moreover, the first curved surface and the second curved surface are configured such that when the joint connector pair swings to the condition that the second partial surface 116 is in contact with the fourth partial surface 126 on the same side, a contact portion is formed between the second partial surface 116 and the fourth partial surface 126, and a plane which is tangent to each of the first curved surface and the second curved surface is formed at the contact portion. That is, the plane is the common tangent plane of the first curved surface and the second curved surface, and the first axis passes through the common tangent plane. Further, the angle between the common tangent plane and the first partial surface 115 is greater than 0° and smaller than or equal to 45°, and the angle between the common tangent plane and the third partial surface 125 is greater than 0° and smaller than or equal to 45°. Preferably, the angle between the common tangent plane and the first partial surface 115 is greater than 0° and smaller than or equal to 22.5°, and the angle between the common tangent plane and the third partial surface 125 is greater than 0° and smaller than or equal to 22.5°. In this case, the swing angle between the first joint connector 11 and the second joint connector 12 in each joint connector pair 10 is 0-90°, preferably 0-45°. The swing angle is measured by the angle between the first surface and the second surface. If the first surface includes the first partial surface 115 and the second partial surface 116, with the second surface including the third partial surface 125 and the fourth partial surface 126, then the swing angle is measured by the angle between the first partial surface 115 and the third partial surface 125. That is, when the snake-like joint is placed, the first partial surface 115 and the third partial surface 125 are configured in the horizontal plane, and the second partial surface 116 and the fourth partial surface 126 are both configured as oblique planes. By configuring the second partial surface 116 and the fourth partial surface 126 to be oblique planes, the first joint connector 11 at the proximal end of one joint connector pair 10 is able to partially support the second joint connector 12 at the distal end of the one joint connector pair, so as to ensure the reliability of the snake-like joint for a surgical robot. On the other hand, by adjusting the angle between the second partial surface 116 and the first partial surface 115 as well as the angle between the fourth partial surface 126 and the third partial surface 125, the swing angle between the joint connector pairs 10 is able to be adjusted.

As shown in FIG. 4 and FIG. 5, in one exemplary embodiment, first axial through holes 117 are disposed in the circumference of the first base 110, and the first axial through holes 117 are provided for the flexible structure to extend through. Second axial through holes 127 are disposed in a circumference of the second base 120, and the second axial through holes 127 are provided for the flexible structure to extend through. Further, there are at least two first axial through holes 117 and at least two second axial through holes 127, and positions of at least two of the first axial through holes 117 correspond to those of at least two of the axial through holes 127. Preferably, the first axial through holes 117 have one-to-one correspondence to the second axial through holes 127 in number and position. Further, the distribution of the first axial through holes 117 on the first base 110 and the distribution of the second axial through holes 127 on the second base 120 may refer to FIG. 4 and FIG. 5. Specifically, the first axial through holes 117 and the second axial through holes 127 are each uniformly and circumferentially arranged in groups, wherein each of axial through holes groups includes at least one axial through hole. As shown in FIG. 4, each of first axial through holes groups includes only one first axial through hole 117, and each of second axial through holes groups includes only one second axial through hole 127. As shown in FIG. 5, each of first axial through holes groups includes three first axial through holes 117, and each of second axial through holes groups includes three second axial through holes 127. Preferably, the first axial through holes 117 in each of first axial through holes groups are uniformly arranged, and the second axial through holes 127 in each of second axial through holes groups are uniformly arranged.

In one exemplary embodiment, the flexible structure is a metal wire. For the structure of the base (i.e., the first base 110 or the second base 120) shown in FIG. 4, if two metal wires are used to control the swing of one joint connector pair, the two metal wires pass through the through holes (i.e., the first axial through holes 117 or the second axial through holes 127) in different through holes groups, and the two through holes groups are distributed on different sides of the first axis of the joint connector pair. For the structure of the base (i.e., the first base 110 or the second base 120) shown in FIG. 5, the metal wires are also configured as such. The use of more metal wires for the control of swings of one joint connector pair is able to improve the reliability of the one joint connector pair and would not appear the failure of the one joint connector pair due to the broken of one metal wire. In other exemplary embodiments, the swings of one joint connector pair can also be controlled by four or six metal wires.

Further, the snake-like joint for a surgical robot includes a plurality of joint connector pairs 10, e.g., any natural number from two to ten, such as, five, six, eight, or ten. The joint connector pairs 10 are detachably or fixedly connected in sequence. Preferably, the first fastening mechanism 118 is disposed on the third surface of the first base 110, the third surface being opposite to the first surface, and the second fastening mechanism 128 is disposed on the fourth surface of the second base 120, the fourth surface being opposite to the second surface, wherein the second fastening mechanism 128 of one joint connector pair is lockable with the first fastening mechanism 118 of the adjacent joint connector pair 10, so that the second joint connector 12 of the one joint connector pair is fixed to the first joint connector 11 of the adjacent joint connector pair 10. In other exemplary embodiments, the second joint connector 12 of one joint connector pair and the first joint connector 11 of the adjacent joint connector pair 10 may also be fixedly connected in other manners, such as an integral forming manner.

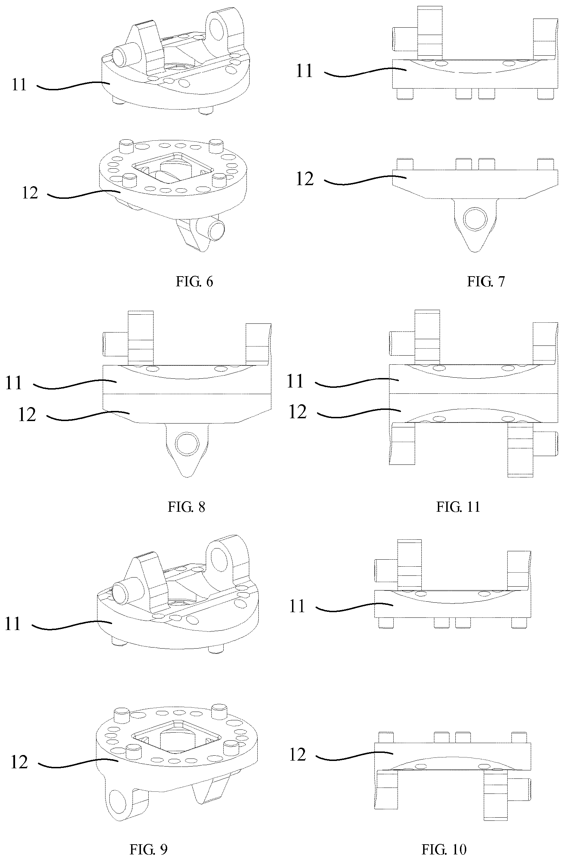

Specifically, as shown in FIG. 6 and FIG. 7, the fastening mechanism includes a boss engaged with a recess in the adjacent fastening mechanism, and a recess housing the boss in the adjacent fastening mechanism. The distribution of the boss and the recess are not particularly limited. In one embodiment, the first fastening mechanism 118 comprises a plurality of first bosses and a plurality of first recesses, the first bosses and the first recesses being arranged at intervals, and the second fastening mechanism 128 comprises a plurality of second bosses and a plurality of second recesses, the second bosses and the second recesses being arranged at intervals, wherein the first bosses correspond to the second recesses in shape, number and position, and the second bosses correspond to the first recesses in shape, number and position.

Further, different swing directions of the joint connector pairs 10 are able to be achieved through different engagements between the joint connector pairs 10, thereby resulting in multiple degrees of freedom of the snake-like joint for a surgical robot.

Specifically, the angle between the third axis of the second joint connector 12 at the proximal end of adjacent joint connector pairs 10 and the first axis of the first joint connector 11 at the distal end of adjacent joint connector pairs 10 is changed, so that the circumferential offset angles of adjacent joint connector pairs are different, which means that adjacent joint connector pairs swing with different directions. Preferably, the circumferential offset angle of adjacent joint connector pairs 10 is greater than or equal to 0° and smaller than or equal to 180°.

Then, referring to FIG. 6-FIG. 8 and FIG. 9-FIG. 11, which are schematic structural views of the second joint connector in one joint connector pair and the adjacent first joint connector in adjacent joint connector pair according to an embodiment, and only show the second joint connector 12 in the proximal joint connector pair and the first joint connector 11 in the distal joint connector pair. FIG. 6-FIG. 8 show the case that the circumferential offset angle of adjacent joint connector pairs is 90°. In this case, the adjacent joint connector pairs are able to swing towards two directions, so that the snake-like joint for a surgical robot has two degrees of freedom. FIG. 9-FIG. 11 show the case that no circumferential offset is existed in adjacent joint connector pairs, so the circumferential offset angle is 0°. In this case, the adjacent joint connector pairs can swing towards one direction with a larger angle (that is, the swing angle is larger than that of one joint connector pair), and the snake-like joint for a surgical robot has only one degree of freedom.

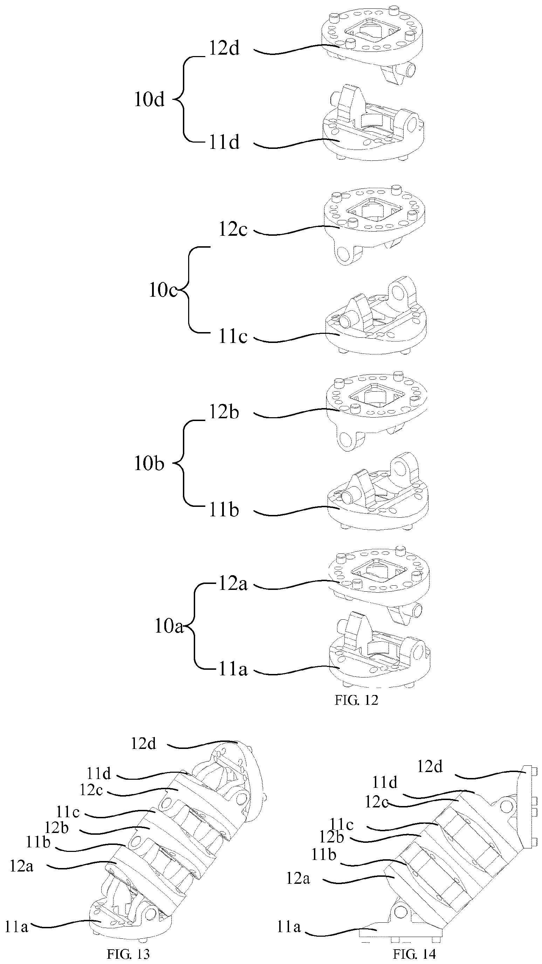

Next, referring to FIG. 1-FIG. 3 and FIG. 12-FIG. 14 simultaneously, FIG. 12-FIG. 14 are schematic structural views of a plurality of joint connector pairs of the snake-like joint for a surgical robot according to an embodiment of the present application. Compared with the previous embodiment, the snake-like joint for a surgical robot in this embodiment having two degrees of freedom includes four joint connector pairs 10 as shown in FIG. 1-FIG. 3, which are the first joint connector pair 10a, the second joint connector pair 10b, the third joint connector pair 10c, and the fourth joint connector pair 10d from the proximal end to the distal end (in other words, from bottom to top). The circumferential offset angle between the second joint connector pair 10b and the first joint connector pair 10a is 90°, the circumferential offset angle between the third joint connector pair 10c and the second joint connector pair 10b is 0° (i.e., parallel to each other), and the circumferential offset angle between the fourth joint connector pair 10d and the third joint connector pair 10c is 90°. In FIG. 12-FIG. 14, the swing direction of the second joint connector pair 10b is the same as that of the third joint connector pair 10c, and the swing direction of the first joint connector pair 10a is the same as that of the fourth joint connector pair 10d, the swing directions of the second joint connector pair 10b and the third joint connector pair 10c being perpendicular to the swing directions of the first joint connector pair 10a and the fourth joint connector pair 10d. Accordingly, the snake-like joint for a surgical robot has two degrees of freedom. In this case, by configuring relative positions of the joint connector pairs 10, it is possible for the snake-like joints to have multiple degrees of freedom, to implement planar torsion or spatial torsion of the snake-like joints.

Further, in FIG. 12-FIG. 14, the maximum swing angle of each joint connector pair (i.e., the first joint connector pair 10a, the second joint connector pair 10b, the third joint connector pair 10c, and the fourth joint connector pair 10d) is 45°. In this case, by configuring two joint connector pairs 10 to swing towards the same direction, it is possible to achieve the maximum swing angle of 90° in each swing direction. Here, the maximum swing angle of 90° in each swing direction through two joint connector pairs 10 instead of one joint connector pairs 10, make the snake-like joint for a surgical robot having a higher reliability and stability.

In other embodiments, the snake-like joint for a surgical robot may include more joint connector pairs 10, such as five, six, or ten, according to the design requirements such as the degree of freedom and the maximum swing angle. Further, the circumferential offset angle between adjacent joint connector pairs 10 may be 0-180°, such as 0°, 30°, 45°, 60°, 90°, 120°, 150°, or 180°, thereby achieving a plenty of arrangement in spatial torsion.

Then, referring to FIG. 12-FIG.14, specifically, the first joint connector pair 10a at the proximal end and the fourth joint connector pair 10d at the distal end are controlled by two metal wires, and the second joint connector pair 10b at the proximal end and the third joint connector pair 10c at the distal end are controlled by another two metal wires. In the embodiment, one end of each of two metal wires controlling the first joint connector pair 10a and the fourth joint connector pair 10d is fixed to the second joint connector 12d of the fourth joint connector pair 10d, and one end of each of two metal wires controlling the second joint connector pair 10b and the third joint connector pair 10c may be fixed to the second joint connector 12c of the third joint connector pair 10c, or may be fixed to the second joint connector 12d of the fourth joint connector pair 10d. Therefore, it is possible for four metal wires to control the swing of the snake-like joint for a surgical robot having two degrees of freedom, i.e., to achieve the spatial torsion of the snake-like joint for a surgical robot. Certainly, those skilled in the art should understand that there may be more metal wires in this embodiment. For example, the number of metal wires is eight, and every two metal wires control a joint connector pair, so as to control the swing angle of the snake-like joint more accurately.

Further, the snake-like joint has n degree(s) of freedom (n is a natural number greater than or equal to 1), that is, when the joint connector pair in the snake-like joint can swing with n swing directions, the number of joint connector pairs is greater than or equal to n, and the number of flexible structures is at least 2n. On this basis, the snake-like joint allows to increase the number of flexible structures (for example, each of the joint connector pairs in the same swing direction is controlled by two flexible structures) to control the swing of the joint connector pair more accurately.

Further, the flexible structure controlling the swing direction of one joint connector pair may be fixed to the second joint connector of the one joint connector pair, or to the second joint connector of the joint connector pair located at the distal end of the one joint connector pair (if any). Therefore, the distal ends of at least two flexible structures are fixed to the second joint connector of the furthest joint connector pair of all joint connector pairs. More preferably, one end of each flexible structure is fixed to the second joint connector of the furthest joint connector pair of the plurality of joint connector pairs. In this case, the metal wire is fixed to the second end face of the second joint connector of the furthest joint connector pair after passing through the first axial through hole and the second axial through hole on each joint connector pair, so as to achieve the swing (motion) control of the snake-like joint for a surgical robot through the stretching of a wire rope.

Further, the number of the first axial through holes 117 and the number of the second axial through holes 127 on a joint connector pair may be the same or different. Similarly, the number of the first axial through holes on the different joint connector pairs in the snake-like joint may be the same or different, and the number of the second axial through holes may also be the same or different. However, in this case, the snake-like joint is required to provide at least two channels for the flexible structure to extend through. Specifically, in one joint connector pair, at least two of the first axial through holes 117 correspond to at least two of the second axial through holes 127 in position, so as to make the flexible structure controlling the joint connector pair extend through. Moreover, at least all joint connector pairs located at the proximal end of the one joint connector pair are provided with first axial through holes 117 and second axial through holes 127 at corresponding positions, to form the channel described above for the flexible structure to extend through. That is, the first axial through holes 117 in the one joint connector pair also corresponds to the second axial through holes 127 in the adjacent joint connector pair at the proximal end of the one joint connector pair in position, and the second axial through holes 127 in the adjacent joint connector pair at the proximal end of the one joint connector pair corresponds to the first axial through holes 117 in the same joint connector pair in position, and so on. Preferably, the joint connector pairs at the distal end of the one joint connector pair is provided with the first axial through holes 117 and the second axial through holes 127 at corresponding positions to form the distal extension of the channel described above for the flexible structure to extend through (that is, in this case, the flexible structure is fixed to the joint connector pair at the distal end).

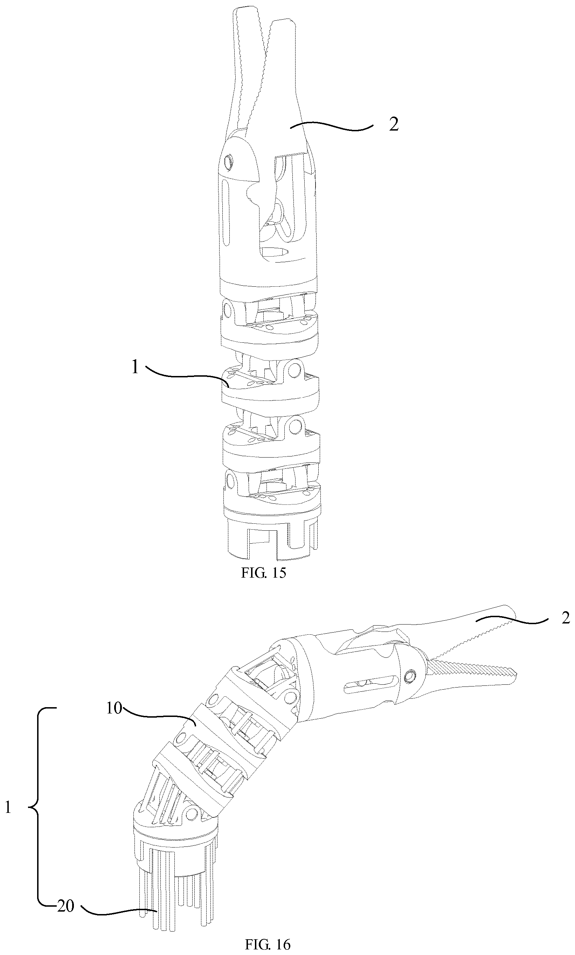

In another exemplary embodiment, it also provides a surgical instrument. As shown in FIG. 15 and FIG. 16, the surgical instrument comprises an instrument terminal 2, a snake-like joint 1 for a surgical robot (the snake-like joint 1 for a surgical robot comprises a plurality of joint connector pairs 10 and a flexible structure 20), a tubular member, a flexible member, and a controller, wherein the instrument terminal, the snake-like joint for a surgical robot, the tubular member, and the controller are sequentially connected from distal end to proximal end, one end of the flexible member being connected to the controller with the other end of the flexible member being connected to the instrument terminal after passing through the tubular member, a proximal end of the flexible structure of the snake-like joint for a surgical robot being connected to the controller after passing through the tubular member, the controller controlling the movement of the instrument terminal through the flexible member, and controlling movements of the snake-like joint for a surgical robot through the flexible structure of the snake-like joint for a surgical robot. Specifically, the instrument terminal mainly includes end-effectors such as scissors, pliers, and an electric hook, which are directly operated in a human body. The distal end of the snake-like joint for a surgical robot is connected to the instrument terminal, while the proximal end is connected to the tubular member. The tubular member is hollow thin-walled tube for supporting the instrument terminal, so that the instrument terminal is able to extend into the human body with its proximal end disposed outside the body. The controller is used for controlling movements of the instrument terminal and the snake-like joint of a surgical robot. One end of the flexible member is connected to the controller, and the other end is connected to the instrument terminal after passing through the tubular member. The proximal end of the flexible structure of the snake-like joint for a surgical robot is connected to the controller after passing through the tubular member. The surgical instrument brings the instrument terminal to a desired position and posture by controlling the posture of the snake-like joint of a surgical robot, and enables the instrument terminal to perform clamping, cutting, and other actions by controlling the instrument terminal.

Further, in still another embodiment, it also provides an endoscope, comprising: an imaging system, a snake-like joint for a surgical robot, a tubular member, and a controller, wherein the imaging system, the snake-like joint for a surgical robot, the tubular member, and the controller are sequentially connected from distant end to proximal end, a proximal end of the flexible structure of the snake-like joint for a surgical robot being connected to the controller after passing through the tubular member, the controller controlling movements of the snake-like joint for a surgical robot through the flexible structure of the snake-like joint for a surgical robot. The imaging system includes an objective lens of the endoscope for acquiring pictures in an endoscopic field of view. The snake-like joint for a surgical robot is used for connecting the imaging system and the tubular member to adjust the posture of the imaging system. The tubular member is a hollow thin-walled tube for supporting the endoscope, so that the distal end of the endoscope is able to extend into the human body with the proximal end of the endoscope disposed outside the body. The controller is used for controlling movements of the snake-like joint for a surgical robot. The proximal end of the flexible structure of the snake-like joint for a surgical robot is connected to the controller after passing through the tubular member. By the control of the posture of the snake-like joint of a surgical robot through the controller, the imaging system of the endoscope can reach a desired posture.

In summary, the snake-like joint for a surgical robot provided in exemplary embodiments achieves the planar torsion or spatial torsion through swings of the joint connector pair, enabling to reduce the complexity of structure thereof compared to the snake-like joint in the prior art. The surgical instrument and the endoscope provided in other exemplary embodiments include the snake-like joint for a surgical robot, thereby enabling the instrument terminal or the imaging system to reach a desired position and posture.

The above description is only a description of the preferred embodiments of the present application, and is not intended to limit the scope of the present application.

**Claims**

1. A snake-like joint for a surgical robot (1), wherein the snake-like joint has at least one degree of freedom, and comprises at least one joint connector pair (10, 10a, 10b, 10c, 10d) and a flexible structure, wherein,

   each joint connector pair (10, 10a, 10b, 10c, 10d) comprises a first joint connector (11, 11a, 11b, 11c, 11d) at a lower end and a second joint connector (12, 12a, 12b, 12c, 12d) at an upper end;
   wherein the first joint connector (11, 11a, 11b, 11c, 11d) comprises a first base as well as a first support structure (111) and a second support structure (112) disposed on a first surface of the first base, the first support structure (111) having a first protrusion (113) formed thereon, the second support structure (112) having a first connecting hole (114) formed therein;
   wherein the second joint connector (12, 12a, 12b, 12c, 12d) comprises a second base as well as a third support structure (121) and a fourth support structure (122) disposed on a second surface of the second base, the third support structure (121) having a second protrusion (123) formed thereon, the fourth support structure (122) having a second connecting hole (124) formed therein;
   when the first joint connector (11, 11a, 11b, 11c, 11d) and the second joint connector (12, 12a, 12b, 12c, 12d) are engaged with each other, the first surface and the second surface are disposed opposite to each other, the first protrusion (113) being configured to locate in the second connecting hole (124) and rotate relative to the second connecting hole (124), the second protrusion (123) being configured to locate in the first connecting hole (114) and rotate relative to the first connecting hole (114);
   the flexible structure controls the joint connector pair (10, 10a, 10b, 10c, 10d) to swing around a joint axis,
   **characterized in that** : the first protrusion (113) is disposed on a first side of the first support structure (111), the first side being a side of the first support structure (111) away from the axis of the snake-like joint;
   a side of the first support structure (111) close to the axis of the snake-like joint is a fifth side;
   the second protrusion (123) is disposed on a second side of the third support structure (121), the second side being a side of the third support structure (121) away from the axis of the snake-like joint;
   a side of the third support structure (121) close to the axis of the snake-like joint

is a sixth side;

a side of the second support structure (112) close to the axis of the snake-like joint is a third side;

a side of the fourth support structure (122) close to the axis of the snake-like joint is a fourth side;

a distance 11 between the first side and the third side and a distance 12 between the second side and the fourth side have the following relationship:

l1-lp2<l2<l1+lp1,

or,

l2-lp1<l1<l2+lp2,

wherein lp1 is an axial length of the first protrusion (113), and lp2 is an axial length of the second protrusion (123);

and,

a sum of the axial length of the first protrusion (113) and a distance between the first side and the fifth side is smaller than a distance between the fourth side and the sixth side;

a sum of the axial length of the second protrusion (123) and a distance between the second side and the sixth side is smaller than a distance between the third side and the fifth side; or,

a side of the second support structure close to the axis of the snake-like joint is a third side;

a side of the fourth support structure close to the axis of the snake-like joint is a fourth side;

the first protrusion (113) is located on a fifth side of the first support structure (111), the fifth side being a side of the first support structure close to the axis of the snake-like joint;

the second protrusion (123) is located on a sixth side of the third support structure (121), the sixth side being a side of the third support structure close to the axis of the snake-like joint;

a side of the second support structure (112) away from the axis of the snake-like joint is a seventh side;

a side of the fourth support structure (122) away from the axis of the snake-like joint is an eighth side;

a distance 13 between the fifth side and the seventh side and a distance 14 between the sixth side and the eighth side have the following relationship:

l3-lp1<l4<l3+lp2,

or,

l4-lp2<l3<l4+lp1,

wherein lp1 is an axial length of the first protrusion (113), and lp2 is an axial length of the second protrusion (123);

and,

a distance between the fourth side and the eighth side is smaller than a difference of a distance between the third side and the fifth side and the axial length of the first protrusion (113);

a distance between the third side and the seventh side is smaller than a difference of a distance between the fourth side and the sixth side and the axial length of the second protrusion (123).

2. The snake-like joint for a surgical robot (1) according to claim 1, wherein the first protrusion (113) has a revolving body structure formed by a straight line or a curve around a first axis, and the first connecting hole (114) has a revolving surface structure formed by a straight line or a curve around a second axis, each of the first axis and the second axis being vertically intersected with the axis of the snake-like joint;

wherein the second protrusion (123) has a revolving body structure formed by a straight line or a curve around a third axis, and the second connecting hole (124) has a revolving surface structure formed by a straight line or a curve around a fourth axis; and

when the first joint connector (11, 11a, 11b, 11c, 11d) and the second joint connector (12, 12a, 12b, 12c, 12d) are engaged with each other, the joint axis, the first axis, the second axis, the third axis, and the fourth axis are collinear, preferably, each of the first protrusion (113) and the second protrusion (123) has a structure of a cylinder, a circular truncated cone, or a circular cone; and each of the first connecting hole (114) and the second connecting hole (124) has a structure of a cylindrical surface, a circular truncated cone surface, or a circular conical surface.

3. The snake-like joint for a surgical robot (1) according to claim 1, wherein

a sum of the axial length of the first protrusion (113) and a distance from the first side of the first support structure (111) to a seventh side of the second support structure (112) is smaller than or equal to a diameter of the first base, wherein a side of the second support structure (112) away from the axis of the snake-like joint is the seventh side;

a sum of the axial length of the second protrusion (123) and a distance from the second side of the third support structure (121) to an eighth side of the fourth support structure (122) is smaller than or equal to a diameter of the second base, wherein a side of the fourth support structure (122) away from the axis of the snake-like joint is the eighth side;
in the joint connector pair (10, 10a, 10b, 10c, 10d), a distance from an outer end face of the first protrusion (113) to an outer end face of the second protrusion (123) is smaller than or equal to the diameter of the first base, and the distance from the outer end face of the first protrusion (113) to the outer end face of the second protrusion (123) is also smaller than or equal to the diameter of the second base.

4. The snake-like joint for a surgical robot (1) according to claim 1, wherein

a distance from a first side of the first support structure (111) to the seventh side of the second support structure (112) is smaller than or equal to a diameter of the first base, wherein the first side is a side of the first support structure (111) away from the axis of the snake-like joint ;
a distance from a second side of the third support structure (121) to the eighth side of the fourth support structure (122) is smaller than or equal to a diameter of the second base, wherein the second side is a side of the third support structure (121) away from the axis of the snake-like joint ;
in the joint connector pair (10, 10a, 10b, 10c, 10d), a distance from the first side to the second side is smaller than or equal to the diameter of the first base, and in the joint connector pair (10, 10a, 10b, 10c, 10d), the distance from the first side to the second side is also smaller than or equal to the diameter of the second base.

5. The snake-like joint for a surgical robot (1) according to claim 3 or 4, wherein a thickness of the first support structure (111), a thickness of the second support structure (112), and the axial length of the first protrusion (113) are equal; and a thickness of the third support structure (121), a thickness of the fourth support structure (122), and the axial length of the second protrusion (123) are equal.

6. The snake-like joint for a surgical robot (1) according to claim 1, wherein the first surface comprises a first partial surface (115) and a second partial surface (116) intersected with the first partial surface (115), each of the first support structure (111) and the second support structure (112) being disposed on the first partial surface (115), and wherein the second surface comprises a third partial surface (125) and a fourth partial surface (126) intersected with the third partial surface (125), each of the third support structure (121) and the fourth support structure (122) being disposed on the third partial surface (125).

7. The snake-like joint for a surgical robot (1) according to claim 6, wherein

the second partial surface (116) is flat, and the joint axis locates in a plane where the second partial surface (116) is located;
the fourth partial surface (126) is flat, and the joint axis locates in a plane where the fourth partial surface (126) is located,
preferably,
an angle between the second partial surface (116) and the first partial surface (115) is 0°-45°, and an angle between the fourth partial surface (126) and the third partial surface (125) is 0°-45°.

8. The snake-like joint for a surgical robot (1) according to claim 6, wherein

the second partial surface (116) and the fourth partial surface (126) are both curved surfaces configured such that: when the joint connector pair (10, 10a, 10b, 10c, 10d) swings to a condition that the second partial surface (116) is in contact with the fourth partial surface (126) on a same side, a contact portion is formed between the second partial surface (116) and the fourth partial surface (126), and a common tangent plane is present between the curved surfaces at the contact portion with the first axis passing through the common tangent plane,
preferably,
an angle between the common tangent plane and the first partial surface (115) is greater than 0° and smaller than or equal to 45°, and an angle between the common tangent plane and the third partial surface (125) is greater than 0° and smaller than or equal to 45°.

9. The snake-like joint for a surgical robot (1) according to claim 1 or 6, wherein a swing angle between the first joint connector (11, 11a, 11b, 11c, 11d) and the second joint connector (12, 12a, 12b, 12c, 12d) in each joint connector pair (10, 10a, 10b, 10c, 10d) is greater than 0° and smaller than or equal to 90°.

10. The snake-like joint for a surgical robot (1) according to claims 1 or 2, wherein first axial through holes (117) are disposed in a circumference of the first base, and the first axial through holes (117) are provided for the flexible structure to extend through; wherein second axial through holes (127) are disposed in a circumference of the second base, and the second axial through holes (127) are provided for the flexible structure to extend through.

11. The snake-like joint for a surgical robot (1) according to claim 10, wherein a number of the first axial through holes (117) and a number of the second axial through holes (127) are plural, and positions of at least two of the first axial through holes (117) correspond to positions of at least two of the second axial through holes (127),
preferably,
in each joint connector pair (10, 10a, 10b, 10c, 10d), the number of the first axial through holes (117) is the same as the number of the second axial through holes (127), and positions of the first axial through holes (117) have a one-to-one correspondence to positions of the second axial through holes (127).

12. The snake-like joint for a surgical robot (1) according to claims 1 or 2, wherein the snake-like joint comprises a plurality of joint connector pairs (10, 10a, 10b, 10c, 10d), and adjacent joint connector pairs are detachably or fixedly connected.

13. The snake-like joint for a surgical robot (1) according to claim 12, wherein a first fastening mechanism (118) is disposed on a third surface of the first base, the third surface being opposite to the first surface; and a second fastening mechanism (128) is disposed on a fourth surface of the second base, the fourth surface being opposite to the second surface; wherein a second fastening mechanism (128) of one joint connector pair (10, 10a, 10b, 10c, 10d) is lockable with a first fastening mechanism (118) of an adjacent joint connector pair, so that the second joint connector (12, 12a, 12b, 12c, 12d) of the one joint connector pair (10, 10a, 10b, 10c, 10d) is detachably connected to the first joint connector (11, 11a, 11b, 11c, 11d) of the adjacent joint connector pair,
preferably,
the first fastening mechanism (118) comprises a plurality of first bosses and a plurality of first recesses, and the first bosses and the first recesses are arranged at intervals; wherein the second fastening mechanism (128) comprises a plurality of second bosses and a plurality of second recesses, and the second bosses and the second recesses are arranged at intervals; wherein the first bosses correspond to the second recesses in shape, number and position, and the second bosses correspond to the first recesses in shape, number and position.

14. A surgical instrument comprising : an instrument terminal (2), a snake-like joint for a surgical robot (1) according to any one of claims 1 to 13, a tubular member, a flexible member, and a controller, wherein the instrument terminal (2), the snake-like joint for a surgical robot (1), the tubular member, and the controller are sequentially connected from distal end to proximal end, one end of the flexible member being connected to the controller with the other end of the flexible member being connected to the instrument terminal (2) after passing through the tubular member, a proximal end of a flexible structure of the snake-like joint for a surgical robot (1) being connected to the controller after passing through the tubular member, the controller controlling movements of the instrument terminal (2) through the flexible member, and controlling swings of the snake-like joint for a surgical robot (1) through the flexible structure of the snake-like joint for a surgical robot (1).

15. An endoscope comprising : an imaging system, a snake-like joint for a surgical robot (1) according to any one of claims 1 to 13, a tubular member, and a controller, wherein the imaging system, the snake-like joint for a surgical robot (1), the tubular member, and the controller are sequentially connected from distal end to proximal end; a proximal end of a flexible structure of the snake-like joint for a surgical robot (1) being connected to the controller after passing through the tubular member, the controller controlling swings of the snake-like joint for a surgical robot (1) through the flexible structure of the snake-like joint for a surgical robot (1), so as to adjust a posture of the imaging system.

**Patentansprüche**

1. Schlangenförmiges Gelenk für einen chirurgischen Roboter (1), wobei das schlangenförmige Gelenk mindestens einen Freiheitsgrad aufweist, und mindestens ein Gelenkverbinderpaar (10, 10a, 10b, 10c, 10d) und eine flexible Struktur umfasst, wobei

jedes Gelenkverbinderpaar (10, 10a, 10b, 10c, 10d) an einem unteren Ende einen ersten Gelenkverbinder (11, 11a, 11b, 11c, 11d) und an einem oberen Ende einen zweiten Gelenkverbinder (12, 12a, 12b, 12c, 12d) umfasst; wobei der erste Gelenkverbinder (11, 11a, 11b, 11c, 11d) eine erste Basis sowie eine erste Stützstruktur (111) und eine zweite Stützstruktur (112) umfasst, die auf einer ersten Fläche der ersten Basis angeordnet sind, wobei die erste Stützstruktur (111) einen darauf ausgebildeten ersten Vorsprung (113) umfasst, wobei die zweite Stützstruktur (112) ein darin ausgebildetes erstes Verbindungsloch (114) umfasst; wobei der zweite Gelenkverbinder (12, 12a, 12b, 12c, 12d) eine zweite Basis sowie eine dritte Stützstruktur (121) und eine vierte Stützstruktur (122) umfasst, die auf einer zweiten Fläche der zweiten Basis angeordnet sind, wobei die dritte Stützstruktur (121) einen darauf ausgebildeten zweiten Vorsprung (123) umfasst, wobei die vierte Stützstruktur (122) ein darin ausgebildetes zweites Verbindungsloch (124) umfasst; wobei, wenn der erste Gelenkverbinder (11, 11a, 11b, 11c, 11d) und der zweite Gelenkverbinder (12, 12a, 12b, 12c, 12d) miteinander in Eingriff stehen, die erste Fläche und die zweite Fläche einander gegenüberliegend angeordnet sind, wobei der erste Vorsprung (113) ausgebildet ist, sich in dem zweiten Verbindungsloch (124) zu befinden und sich relativ zu dem zweiten Verbindungsloch (124) zu drehen, wobei der zweite Vorsprung (123) ausgebildet ist, sich in dem ersten Verbindungsloch (114) zu befinden und sich relativ zu dem ersten Verbindungsloch (114) zu drehen;

wobei die flexible Struktur das Gelenkverbinderpaar (10, 10a, 10b, 10c, 10d) zum Schwingen um eine Gelenkachse steuert,

**dadurch gekennzeichnet, dass** der erste Vorsprung (113) auf einer ersten Seite der ersten Stützstruktur (111) angeordnet ist, wobei die erste Seite eine Seite der ersten Stützstruktur (111) fern von der Achse des schlangenförmigen Gelenks ist;

wobei eine Seite der ersten Stützstruktur (111) nahe der Achse des schlangenförmigen Gelenks eine fünfte Seite ist;

wobei der zweite Vorsprung (123) auf einer zweiten Seite der dritten Stützstruktur (121) angeordnet ist, wobei die zweite Seite eine Seite der dritten Stützstruktur (121) fern von der Achse des schlangenförmigen Gelenks ist;

wobei eine Seite der dritten Stützstruktur (121) nahe der Achse des schlangenförmigen Gelenks eine sechste Seite ist;

wobei eine Seite der zweiten Stützstruktur (112) nahe der Achse des schlangenförmigen Gelenks eine dritte Seite ist;

wobei eine Seite der vierten Stützstruktur (122) nahe der Achse des schlangenförmigen Gelenks eine vierte Seite ist;

wobei ein Abstand l1 zwischen der ersten Seite und der dritten Seite und ein Abstand l2 zwischen der zweiten Seite und der vierten Seite den folgenden Zusammenhang aufweist:

$$l1 - lp2 < l2 < l1 + lp1,$$

oder,

$$l2 - lp1 < l1 < l2 + lp2,$$

wobei lp1 eine axiale Länge des ersten Vorsprungs (113) ist, und wobei lp2 eine axiale Länge des zweiten Vorsprungs (123) ist; und,

wobei eine Summe aus der axialen Länge des ersten Vorsprungs (113) und einem Abstand zwischen der ersten Seite und der fünften Seite kleiner als ein Abstand zwischen der vierten Seite und der sechsten Seite ist;

wobei eine Summe aus der axialen Länge des zweiten Vorsprungs (123) und einem Abstand zwischen der zweiten Seite und der sechsten Seite kleiner als ein Abstand zwischen der dritten Seite und der fünften Seite ist;

oder,

wobei eine Seite der zweiten Stützstruktur nahe der Achse des schlangenförmigen Gelenks eine dritte Seite ist;

wobei eine Seite der vierten Stützstruktur nahe der Achse des schlangenförmigen Gelenks eine vierte Seite ist;

wobei der erste Vorsprung (113) auf einer fünften Seite der ersten Stützstruktur (111) angeordnet ist, wobei die fünfte Seite eine Seite der ersten Stützstruktur nahe der Achse des schlangenförmigen Gelenks ist;

wobei der zweite Vorsprung (123) auf einer sechsten Seite der dritten Stützstruktur (121) angeordnet ist, wobei die sechste Seite eine Seite der dritten Stützstruktur nahe der Achse des schlangenförmigen Gelenks ist;

wobei eine Seite der zweiten Stützstruktur (112) fern von der Achse des schlangenförmigen Gelenks eine siebte Seite ist;

wobei eine Seite der vierten Stützstruktur (122) fern von der Achse des schlangenförmigen Gelenks eine achte Seite ist;

wobei ein Abstand l3 zwischen der fünften Seite und der siebten Seite und ein Abstand l4 zwischen der sechsten Seite und der achten Seite den folgenden Zusammenhang aufweist:

$$l3 - lp1 < l4 < l3 + lp2,$$

oder,

$$l4 - lp2 < l3 < l4 + lp1,$$

wobei lp1 eine axiale Länge des ersten Vorsprungs (113) ist, und lp2 eine axiale Länge des zweiten Vorsprungs (123) ist; und,

wobei ein Abstand zwischen der vierten Seite und der achten Seite kleiner als eine Differenz eines Abstands zwischen der dritten Seite und der fünften Seite und der axialen Länge des ersten Vorsprungs (113) ist;

wobei ein Abstand zwischen der dritten Seite und der siebten Seite kleiner als eine Differenz eines Abstands zwischen der vierten Seite und der sechsten Seite und der axialen Länge des zweiten Vorsprungs (123) ist.

2. Schlangenförmiges Gelenk für einen chirurgischen Roboter (1) nach Anspruch 1, wobei der erste Vorsprung (113) eine umlaufende Körperstruktur aufweist, die durch eine gerade Linie oder eine Kurve um eine erste Achse gebildet ist, und wobei das erste Verbindungsloch (114) eine umlaufende Flächenstruktur aufweist, die durch eine gerade Linie oder eine Kurve um eine zweite Achse herum gebildet ist, wobei jede der ersten Achse als auch der zweiten Achse sich vertikal mit der Achse des schlangenförmigen Gelenks schneiden;

> wobei der zweite Vorsprung (123) eine umlaufende Körperstruktur aufweist, die durch eine gerade Linie oder eine Kurve um eine dritte Achse gebildet ist, und wobei das zweite Verbindungsloch (124) eine umlaufende Flächenstruktur aufweist, die durch eine gerade Linie oder eine Kurve um eine vierte Achse gebildet wird; und
> wobei, wenn der erste Gelenkverbinder (11, 11a, 11b, 11c, 11d) und der zweite Gelenkverbinder (12, 12a, 12b, 12c, 12d) miteinander in Eingriff stehen, die Gelenkachse, die erste Achse, die zweite Achse, die dritte Achse und die vierte Achse kollinear sind,
> wobei, vorzugsweise, jeder des ersten Vorsprungs (113) als auch des zweiten Vorsprungs (123) eine Struktur eines Zylinders, eines kreisförmigen Kegelstumpfes oder eines kreisförmigen Kegels aufweist; und wobei jedes des ersten Verbindungslochs (114) und des zweiten Verbindungslochs (124) eine Struktur einer zylindrischen Fläche, einer kreisförmigen Kegelstumpffläche oder einer kreisförmigen konischen Fläche aufweist.

3. Schlangenförmiges Gelenk für einen chirurgischen Roboter (1) nach Anspruch 1, wobei

> eine Summe aus der axialen Länge des ersten Vorsprungs (113) und einem Abstand von der ersten Seite der ersten Stützstruktur (111) zu einer siebten Seite der zweiten Stützstruktur (112) kleiner oder gleich einem Durchmesser der ersten Basis ist, wobei eine Seite der zweiten Stützstruktur (112) fern von der Achse des schlangenförmigen Gelenks die siebte Seite ist;
> wobei eine Summe aus der axialen Länge des zweiten Vorsprungs (123) und einem Abstand von der zweiten Seite der dritten Stützstruktur (121) zu einer achten Seite der vierten Stützstruktur (122) kleiner oder gleich einem Durchmesser der zweiten Basis ist, wobei eine Seite der vierten Stützstruktur (122) fern von der Achse des schlangenförmigen Gelenks die achte Seite ist;

> wobei in dem Gelenkverbinderpaar (10, 10a, 10b, 10c, 10d) ein Abstand von einer äußeren Endfläche des ersten Vorsprungs (113) zu einer äußeren Endfläche des zweiten Vorsprungs (123) kleiner oder gleich dem Durchmesser der ersten Basis ist, und wobei der Abstand von der äußeren Endfläche des ersten Vorsprungs (113) zur äußeren Endfläche des zweiten Vorsprungs (123) ebenfalls kleiner oder gleich dem Durchmesser der zweiten Basis ist.

4. Schlangenförmiges Gelenk für einen chirurgischen Roboter (1) nach Anspruch 1, wobei

> ein Abstand von einer ersten Seite der ersten Stützstruktur (111) zu der siebten Seite der zweiten Stützstruktur (112) kleiner oder gleich einem Durchmesser der ersten Basis ist, wobei die erste Seite eine Seite der ersten Stützstruktur (111) fern von der Achse der ersten Basis ist;
> wobei ein Abstand von einer zweiten Seite der dritten Stützstruktur (121) zu der achten Seite der vierten Stützstruktur (122) kleiner oder gleich einem Durchmesser der zweiten Basis ist, wobei die zweite Seite eine Seite der dritten Stützstruktur (121) fern von der Achse des schlangenförmigen Gelenks ist;
> wobei in dem Gelenkverbinderpaar (10, 10a, 10b, 10c, 10d) ein Abstand von der ersten Seite zur zweiten Seite kleiner oder gleich dem Durchmesser der ersten Basis ist, und wobei in dem Gelenkverbinderpaar (10, 10a, 10b, 10c, 10d) der Abstand von der ersten Seite zur zweiten Seite ebenfalls kleiner oder gleich dem Durchmesser der zweiten Basis ist.

5. Schlangenförmiges Gelenk für einen chirurgischen Roboter (1) nach Anspruch 3 oder 4, wobei eine Dicke der ersten Stützstruktur (111), eine Dicke der zweiten Stützstruktur (112) und die axiale Länge des ersten Vorsprungs (113) gleich sind; und eine Dicke der dritten Stützstruktur (121), eine Dicke der vierten Stützstruktur (122) und die axiale Länge des zweiten Vorsprungs (123) gleich sind.

6. Schlangenförmiges Gelenk für einen chirurgischen Roboter (1) nach Anspruch 1, wobei die erste Fläche eine erste Teilfläche (115) und eine zweite Teilfläche (116) umfasst, die sich mit der ersten Teilfläche (115) schneidet, wobei jeweils die erste Stützstruktur (111) und die zweiten Stützstruktur (112) auf der ersten Teilfläche (115) angeordnet sind, und wobei die zweite Fläche eine dritte Teilfläche (125) und eine mit der dritten Teilfläche (125) geschnittene vierte Teilfläche (126) umfasst, wobei jeweils die dritte Stützstruktur (121) und die vierte Stützstruktur (122) auf der dritten Teilfläche (125) angeordnet sind.

7. Schlangenförmiges Gelenk für einen chirurgischen Roboter (1) nach Anspruch 6, wobei

die zweite Teilfläche (116) eben ist, und wobei die Gelenkachse in einer Ebene liegt, in der sich die zweite Teilfläche (116) befindet;
wobei die vierte Teilfläche (126) eben ist, und wobei die Gelenkachse in einer Ebene liegt, in der sich die vierte Teilfläche (126) befindet,
wobei vorzugsweise,
ein Winkel zwischen der zweiten Teilfläche (116) und der ersten Teilfläche (115) 0°-45° ist und ein Winkel zwischen der vierten Teilfläche (126) und der dritten Teilfläche (125) 0°-45° ist.

8. Schlangenförmiges Gelenk für einen chirurgischen Roboter (1) nach Anspruch 6, wobei

die zweite Teilfläche (116) und die vierte Teilfläche (126) beide gekrümmte Flächen sind, die so ausgebildet sind, dass: wenn das Gelenkverbinderpaar (10, 10a, 10b, 10c, 10d) in einen Zustand schwenkt, in dem die zweite Teilfläche (116) mit der vierten Teilfläche (126) auf einer gleichen Seite in Kontakt steht, ist ein Kontaktabschnitt zwischen der zweiten Teilfläche (116) und der vierten Teilfläche (126) geformt, und ist eine gemeinsame Tangentialebene zwischen den gekrümmten Flächen am Kontaktabschnitt vorhanden, wobei die erste Achse durch die gemeinsame Tangentialebene verläuft,
wobei vorzugsweise,
ein Winkel zwischen der gemeinsamen Tangentialebene und der ersten Teilfläche (115) größer als 0° und kleiner oder gleich 45° ist, und ein Winkel zwischen der gemeinsamen Tangentialebene und der dritten Teilfläche (125) größer als 0° ist und kleiner oder gleich 45° ist.

9. Schlangenförmiges Gelenk für einen chirurgischen Roboter (1) nach Anspruch 1 oder 6, wobei ein Schwenkwinkel zwischen dem ersten Gelenkverbinder (11, 11a, 11b, 11c, 11d) und dem zweiten Gelenkverbinder (12, 12a, 12b, 12c, 12d) in jedem Gelenkverbinderpaar (10, 10a, 10b, 10c, 10d) größer als 0° und kleiner oder gleich 90° ist.

10. Schlangenförmiges Gelenk für einen chirurgischen Roboter (1) nach Anspruch 1 oder 2, wobei erste axiale Durchgangslöcher (117) in einem Umfang der ersten Basis angeordnet sind, und wobei die ersten axialen Durchgangslöcher (117) zur Hindurcherstreckung der flexiblen Struktur vorgesehen sind; wobei zweite axiale Durchgangslöcher (127) in einem Umfang der zweiten Basis angeordnet sind, und wobei die zweiten axialen Durchgangslöcher (127) zur Hindurcherstreckung der flexiblen Struktur vorgesehen sind.

11. Schlangenförmiges Gelenk für einen chirurgischen Roboter (1) nach Anspruch 10, wobei eine Anzahl der ersten axialen Durchgangslöcher (117) und eine Anzahl der zweiten axialen Durchgangslöcher (127) eine Vielzahl sind, und wobei Positionen von mindestens zwei der ersten axialen Durchgangslöcher (117) Positionen von mindestens zwei der zweiten axialen Durchgangslöcher (127) entsprechen,

wobei vorzugsweise,
in jedem Gelenkverbinderpaar (10, 10a, 10b, 10c, 10d) die Anzahl der ersten axialen Durchgangslöcher (117) gleich der Anzahl der zweiten axialen Durchgangslöcher (127) ist, und Positionen der ersten axialen Durchgangslöcher (117) eine Eins-zu-Eins-Entsprechung zu Positionen der zweiten axialen Durchgangslöcher (127) aufweisen.

12. Schlangenförmiges Gelenk für einen chirurgischen Roboter (1) nach Anspruch 1 oder 2, wobei das schlangenförmige Gelenk eine Vielzahl von Gelenkverbinderpaaren (10, 10a, 10b, 10c, 10d) umfasst und wobei benachbarte Gelenkverbinderpaare lösbar oder fest verbunden sind.

13. Schlangenförmiges Gelenk für einen chirurgischen Roboter (1) nach Anspruch 12, wobei ein erster Befestigungsmechanismus (118) auf einer dritten Fläche der ersten Basis angeordnet ist, wobei die dritte Fläche der ersten Fläche gegenüberliegt; und wobei ein zweiter Befestigungsmechanismus (128) auf einer vierten Fläche der zweiten Basis angeordnet ist, wobei die vierte Fläche der zweiten Fläche gegenüberliegt; wobei ein zweiter Befestigungsmechanismus (128) eines Gelenkverbinderpaars (10, 10a, 10b, 10c, 10d) mit einem ersten Befestigungsmechanismus (118) eines benachbarten Gelenkverbinderpaars verschließbar ist, so dass der zweite Gelenkverbinder (12, 12a, 12b, 12c, 12d) des einen Gelenkverbinderpaars (10, 10a, 10b, 10c, 10d) mit dem ersten Gelenkverbinder (11, 11a, 11b, 11c, 11d) des benachbarten Gelenkverbinderpaars lösbar verbunden ist,

wobei vorzugsweise,
der erste Befestigungsmechanismus (118) eine Vielzahl von ersten Buckeln und eine Vielzahl von ersten Aussparungen umfasst, und wobei die ersten Buckel und die ersten Aussparungen in Intervallen angeordnet sind; wobei der zweite Befestigungsmechanismus (128) eine Vielzahl von zweiten Buckeln und eine Vielzahl zweiter Aussparungen umfasst, und wobei die zweiten Buckel und die zweiten Aussparungen in Intervallen angeordnet sind; wobei die ersten Buckel den zweiten Aussparungen in Form, Anzahl und Position entsprechen und die zweiten Buckel den ersten Aussparungen in Form, Anzahl und Position entsprechen.

14. Chirurgisches Instrument, umfassend einen Instrumentenanschluss (2), ein schlangenförmiges Gelenk für einen chirurgischen Roboter (1) nach einem der Ansprüche 1 bis 13, ein röhrenförmiges Element, ein flexibles Element und eine Steuerung, wobei der Instrumentenanschluss (2), das schlangenförmige Gelenk für einen chirurgischen Roboter (1), das röhrenförmige Element und die Steuerung nacheinander vom distalen Ende zum proximalen Ende verbunden sind, wobei ein Ende des flexiblen Elements mit der Steuerung verbunden ist, wobei das andere Ende des flexiblen Elements nach Hindurchführen durch das röhrenförmige Element mit dem Instrumentenanschluss (2) verbunden ist, wobei ein proximales Ende einer flexiblen Struktur des schlangenförmigen Gelenks für einen chirurgischen Roboter (1) nach Hindurchführen durch das röhrenförmige Element mit der Steuerung verbunden ist, wobei die Steuerung Bewegungen des Instrumentenanschlusses (2) durch das flexible Element steuert und Schwenks des schlangenförmigen Gelenks für einen chirurgischen Roboter (1) durch die flexible Struktur des schlangenförmigen Gelenks für einen chirurgischen Roboter (1) steuert.

15. Endoskop, umfassend ein Abbildungssystem, ein schlangenförmiges Gelenk für einen chirurgischen Roboter (1) nach einem der Ansprüche 1 bis 13, ein röhrenförmiges Element und eine Steuerung, wobei das Abbildungssystem, das schlangenähnliche Gelenk für einen chirurgischen Roboter (1), das röhrenförmige Element und die Steuerung nacheinander vom distalen Ende zum proximalen Ende verbunden sind; wobei ein proximales Ende einer flexiblen Struktur des schlangenförmigen Gelenks für einen chirurgischen Roboter (1) mit der Steuerung nach Hindurchführen durch das röhrenförmige Element verbunden ist, wobei die Steuerung Schwenks des schlangenförmigen Gelenks für einen chirurgischen Roboter (1) durch die flexible Struktur des schlangenförmigen Gelenks für einen chirurgischen Roboter (1) steuert, um eine Stellung des Abbildungssystems einzustellen.

**Revendications**

1. Articulation en forme de serpent pour un robot chirurgical (1), l'articulation en forme de serpent ayant au moins un degré de liberté, et comprenant au moins une paire de raccords d'articulation (10, 10a, 10b, 10c, 10d) et une structure souple, dans laquelle,

chaque paire de raccords d'articulation (10, 10a, 10b, 10c, 10d) comprend un premier raccord d'articulation (11, 11a, 11b, 11c, 11d) à une extrémité inférieure et un second raccord d'articulation (12, 12a, 12b, 12c, 12d) à une extrémité supérieure ;
dans laquelle le premier raccord d'articulation (11, 11a, 11b, 11c, 11d) comprend une première base ainsi qu'une première structure de support (111) et une deuxième structure de support (112) disposées sur une première surface de la première base, la première structure de support (111) ayant une première saillie (113) formée sur celle-ci, la deuxième structure de support (112) ayant un premier trou de raccordement (114) formé en son sein ;
dans laquelle le second raccord d'articulation (12, 12a, 12b, 12c, 12d) comprend une seconde base ainsi qu'une troisième structure de support (121) et une quatrième structure de support (122) disposées sur une seconde surface de la seconde base, la troisième structure de support (121) ayant une seconde saillie (123) formée sur celle-ci, la quatrième structure de support (122) ayant un second trou de raccordement (124) formé en son sein ;
lorsque le premier raccord d'articulation (11, 11a, 11b, 11c, 11d) et le second raccord d'articulation (12, 12a, 12b, 12c, 12d) sont en prise l'un avec l'autre, la première surface et la seconde surface sont disposées à l'opposé l'une de l'autre, la première saillie (113) étant configurée pour se situer dans le second trou de raccordement (124) et tourner par rapport au second trou de raccordement (124), la seconde saillie (123) étant configurée pour se situer dans le premier trou de raccordement (114) et tourner par rapport au premier trou de raccordement (114) ;
la structure souple commande la paire de raccords d'articulation (10, 10a, 10b, 10c, 10d) pour se balancer autour d'un axe d'articulation,
**caractérisée en ce que** : la première saillie (113) est disposée sur un premier côté de la première structure de support (111), le premier côté étant un côté de la première structure de support (111) éloigné de l'axe de l'articulation en forme de serpent ;
un côté de la première structure de support (111) proche de l'axe de l'articulation en forme de serpent est un cinquième côté ;
la seconde saillie (123) est disposée sur un deuxième côté de la troisième structure de support (121), le deuxième côté étant un côté de la troisième structure de support (121) éloigné de l'axe de l'articulation en forme de serpent ;

un côté de la troisième structure de support (121) proche de l'axe de l'articulation en forme de serpent est un sixième côté ;

un côté de la deuxième structure de support (112) proche de l'axe de l'articulation en forme de serpent est un troisième côté ;

un côté de la quatrième structure de support (122) proche de l'axe de l'articulation en forme de serpent est un quatrième côté ;

une distance l1 entre le premier côté et le troisième côté et une distance l2 entre le deuxième côté et le quatrième côté ont la relation suivante :

$$l1 - lp2 < l2 < l1 + lp1,$$

ou,

$$l2 - lp1 < l1 < l2 + lp2,$$

dans lesquelles lp1 est une longueur axiale de la première saillie (113), et lp2 est une longueur axiale de la seconde saillie (123) ;
et,

une somme de la longueur axiale de la première saillie (113) et d'une distance entre le premier côté et le cinquième côté est inférieure à une distance entre le quatrième côté et le sixième côté ;

une somme de la longueur axiale de la seconde saillie (123) et d'une distance entre le deuxième côté et le sixième côté est inférieure à une distance entre le troisième côté et le cinquième côté ;

un côté de la deuxième structure de support proche de l'axe de l'articulation en forme de serpent est un troisième côté ;

un côté de la quatrième structure de support proche de l'axe de l'articulation en forme de serpent est un quatrième côté ;

la première saillie (113) est située sur un cinquième côté de la première structure de support (111), le cinquième côté étant un côté de la première structure de support proche de l'axe de l'articulation en forme de serpent ;

la seconde saillie (123) est située sur un sixième côté de la troisième structure de support (121), le sixième côté étant un côté de la troisième structure de support proche de l'axe de l'articulation en forme de serpent ;

un côté de la seconde structure de support (112) éloigné de l'axe de l'articulation en forme de serpent est un septième côté ;

un côté de la quatrième structure de support (122) éloigné de l'axe de l'articulation en forme de serpent est un huitième côté ;

une distance l3 entre le cinquième côté et le septième côté et une distance l4 entre le sixième côté et le huitième côté ont la relation suivante :

$$l3 - lp1 < l4 < l3 < lp2,$$

ou,

$$l4 - lp2 < l3 < l4 + lp1,$$

dans lesquelles lp1 est une longueur axiale de la première saillie (113), et lp2 est une longueur axiale de la seconde saillie (123) ;
et,

une distance entre le quatrième côté et le huitième côté est inférieure à une différence entre une distance entre le troisième côté et le cinquième côté et la longueur axiale de la première saillie (113) ;

une distance entre le troisième côté et le septième côté est inférieure à une différence entre une distance entre le quatrième côté et le sixième côté et la longueur axiale de la seconde saillie (123).

2. Articulation en forme de serpent pour un robot chirurgical (1) selon la revendication 1, dans laquelle la première saillie (113) a une structure de corps rotatif formée par une ligne droite ou une courbe autour d'un premier axe, et le premier trou de raccordement (114) a une structure de surface rotative formée par une ligne droite ou une courbe autour d'un deuxième axe, chacun du premier axe et du deuxième axe étant verticalement intersecté avec l'axe de l'articulation en forme de serpent ;

dans laquelle la seconde saillie (123) a une structure de corps rotatif formée par une ligne droite ou une courbe autour d'un troisième axe, et le second trou de raccordement (124) a une structure de surface rotative formée par une ligne droite ou une courbe autour d'un quatrième axe ; et

lorsque le premier raccord d'articulation (11, 11a, 11b, 11c, 11d) et le second raccord d'articulation (12, 12a, 12b, 12c, 12d) sont en prise l'un avec l'autre, l'axe d'articulation, le premier axe, le deuxième axe, le troisième axe et le quatrième axe sont colinéaires,

de préférence, chacune de la première saillie (113) et de la seconde saillie (123) a une structure de cylindre, de cône circulaire tronqué ou de cône circulaire ; et chacun du premier trou de raccordement (114) et du second trou de raccordement (124) a une structure d'une surface cylindrique, d'une surface de cône circulaire tronqué ou d'une surface conique circulaire.

**3.** Articulation en forme de serpent pour un robot chirurgical (1) selon la revendication 1, dans laquelle

une somme de la longueur axiale de la première saillie (113) et d'une distance entre le premier côté de la première structure de support (111) et un septième côté de la deuxième structure de support (112) est inférieure ou égale à un diamètre de la première base, dans laquelle un côté de la deuxième structure de support (112) éloigné de l'axe de l'articulation en forme de serpent est le septième côté ;

une somme de la longueur axiale de la seconde saillie (123) et d'une distance entre le second côté de la troisième structure de support (121) et un huitième côté de la quatrième structure de support (122) est inférieure ou égale à un diamètre de la seconde base, dans laquelle un côté de la quatrième structure de support (122) éloigné de l'axe de l'articulation en forme de serpent est le huitième côté ;

dans la paire de raccords d'articulation (10, 10a, 10b, 10c, 10d), une distance entre une face d'extrémité externe de la première saillie (113) et une face d'extrémité externe de la seconde saillie (123) est inférieure ou égale au diamètre de la première base, et la distance entre la face d'extrémité externe de la première saillie (113) et la face d'extrémité externe de la seconde saillie (123) est également inférieure ou égale au diamètre de la seconde base.

**4.** Articulation en forme de serpent pour un robot chirurgical (1) selon la revendication 1, dans laquelle

une distance entre un premier côté de la première structure de support (111) et le septième côté de la seconde structure de support (112) est inférieure ou égale à un diamètre de la première base, dans laquelle le premier côté est un côté de la première structure de support (111) éloigné de l'axe de l'articulation en forme de serpent ;

une distance entre un deuxième côté de la troisième structure de support (121) et le huitième côté de la quatrième structure de support (122) est inférieure ou égale à un diamètre de la seconde base, dans laquelle le deuxième côté est un côté de la troisième structure de support (121) éloigné de l'axe de l'articulation en forme de serpent ;

dans la paire de raccords d'articulation (10, 10a, 10b, 10c, 10d), une distance entre le premier côté et le deuxième côté est inférieure ou égale au diamètre de la première base, et dans la paire de raccords d'articulation (10, 10a, 10b, 10c, 10d), la distance entre le premier côté et le deuxième côté est également inférieure ou égale au diamètre de la seconde base.

**5.** Articulation en forme de serpent pour un robot chirurgical (1) selon la revendication 3 ou 4, dans laquelle une épaisseur de la première structure de support (111), une épaisseur de la deuxième structure de support (112) et la longueur axiale de la première saillie (113) sont égales ; et une épaisseur de la troisième structure de support (121), une épaisseur de la quatrième structure de support (122) et la longueur axiale de la seconde saillie (123) sont égales.

**6.** Articulation en forme de serpent pour un robot chirurgical (1) selon la revendication 1, dans laquelle la première surface comprend une première surface partielle (115) et une seconde surface partielle (116) intersectée avec la première surface partielle (115), chacune de la première structure de support (111) et de la deuxième structure de support (112) étant disposée sur la première surface partielle (115), et dans laquelle la seconde surface comprend une troisième surface partielle (125) et une quatrième surface partielle (126) intersectée avec la troisième surface partielle (125), chacune de la troisième structure de support (121) et de la quatrième structure de support (122) étant disposée sur la troisième surface partielle (125).

**7.** Articulation en forme de serpent pour un robot chirurgical (1) selon la revendication 6, dans laquelle

la deuxième surface partielle (116) est plate, et l'axe d'articulation se situe dans un plan où se trouve la deuxième surface partielle (116) ;

la quatrième surface partielle (126) est plate, et l'axe d'articulation se situe dans un plan où se trouve la quatrième surface partielle (126),

de préférence,

un angle entre la seconde surface partielle (116) et la première surface partielle (115) est de 0° à 45°, et un angle entre la quatrième surface partielle (126) et la troisième surface partielle (125) est de 0° à 45°.

**8.** Articulation en forme de serpent pour un robot chirurgical (1) selon la revendication 6, dans laquelle

la seconde surface partielle (116) et la quatrième surface partielle (126) sont toutes deux des surfaces courbes configurées de telle sorte que : lorsque la paire de raccords d'articulation (10, 10a, 10b, 10c, 10d) bascule vers un état dans lequel la deuxième surface partielle (116) est en contact avec la quatrième surface partielle (126) sur un même côté, une partie de contact soit formée entre la deuxième surface partielle (116) et la quatrième surface partielle (126), et qu'un plan tangent commun soit présent entre les surfaces courbes au niveau de la partie de contact, le premier axe passant par le plan tangent commun,

de préférence,

un angle entre le plan tangent commun et la première surface partielle (115) est supérieur à 0° et inférieur ou égal à 45°, et un angle entre le plan tangent commun et la troisième surface partielle (125) est supérieur à 0° et inférieur ou égal à 45°.

9. Articulation en forme de serpent pour un robot chirurgical (1) selon la revendication 1 ou 6, dans lequel un angle de bascule entre le premier raccord d'articulation (11, 11a, 11b, 11c, 11d) et le second raccord d'articulation (12, 12a, 12b, 12c, 12d) dans chaque paire de raccords d'articulation (10, 10a, 10b, 10c, 10d) est supérieur à 0° et inférieur ou égal à 90°.

10. Articulation en forme de serpent pour un robot chirurgical (1) selon les revendications 1 ou 2, dans laquelle des premiers trous traversants axiaux (117) sont disposés dans une circonférence de la première base, et les premiers trous traversants axiaux (117) sont prévus pour que la structure souple s'étende à travers ; dans laquelle des seconds trous traversants axiaux (127) sont disposés dans une circonférence de la seconde base, et les seconds trous traversants axiaux (127) sont prévus pour que la structure souple s'étende à travers.

11. Articulation en forme de serpent pour un robot chirurgical (1) selon la revendication 10, dans laquelle un nombre des premiers trous traversants axiaux (117) et un nombre des seconds trous traversants axiaux (127) sont pluriels, et des positions d'au moins deux des premiers trous traversants axiaux (117) correspondent aux positions d'au moins deux des seconds trous traversants axiaux (127),

de préférence,

dans chaque paire de raccords d'articulation (10, 10a, 10b, 10c, 10d), le nombre des premiers trous traversants axiaux (117) est le même que le nombre des seconds trous traversants axiaux (127), et les positions des premiers trous traversants axiaux (117) ont une correspondance biunivoque avec les positions des seconds trous traversants axiaux (127).

12. Articulation en forme de serpent pour un robot chirurgical (1) selon les revendications 1 ou 2, l'articulation en forme de serpent comprenant une pluralité de paires de raccords d'articulation (10, 10a, 10b, 10c, 10d), et des paires de raccords d'articulation adjacents sont raccordées de manière détachable ou à demeure.

13. Articulation en forme de serpent pour un robot chirurgical (1) selon la revendication 12, dans laquelle un premier mécanisme de fixation (118) est disposé sur une troisième surface de la première base, la troisième surface étant opposée à la première surface ; et un second mécanisme de fixation (128) est disposé sur une quatrième surface de la seconde base, la quatrième surface étant opposée à la seconde surface ; dans laquelle un second mécanisme de fixation (128) d'une paire de raccords d'articulation (10, 10a, 10b, 10c, 10d) est verrouillable avec un premier mécanisme de fixation (118) d'une paire de raccords d'articulation adjacents, de sorte que le second raccord d'articulation (12, 12a, 12b, 12c, 12d) de la paire de raccords d'articulation (10, 10a, 10b, 10c, 10d) soit raccordé de façon détachable au premier raccord d'articulation (11, 11a, 11b, 11c, 11d) de la paire de raccords d'articulation adjacents,

de préférence,

le premier mécanisme de fixation (118) comprend une pluralité de premières bosses et une pluralité de premiers évidements, et les premières bosses et les premiers évidements sont disposés à intervalles ; dans laquelle le second mécanisme de fixation (128) comprend une pluralité de secondes bosses et une pluralité de seconds évidements, et les secondes bosses et les seconds évidements sont disposés à intervalles ; dans laquelle les premières bosses correspondent aux seconds évidements en ce qui concerne la forme, le nombre et la position, et les secondes bosses correspondent aux premiers évidements en ce qui concerne la forme, le nombre et la position.

14. Instrument chirurgical comprenant un terminal (2) d'instrument, une articulation en forme de serpent pour un robot chirurgical (1) selon l'une quelconque des revendications 1 à 13, un élément tubulaire, un élément souple et un organe de commande, dans lequel le terminal (2) d'instrument, l'articulation en forme de serpent pour un robot chirurgical (1), l'élément tubulaire et l'organe de commande sont raccordés de façon séquentielle de l'extrémité distale à l'extrémité proximale, une extrémité de l'élément souple étant raccordée à l'organe de commande, l'autre extrémité de l'élément souple étant raccordée au terminal (2) d'instrument après avoir traversé l'élément tubulaire, une extrémité proximale d'une structure souple de l'articulation en forme de serpent pour un robot chirurgical (1) étant raccordée à l'organe de commande après avoir traversé l'élément tubulaire, l'organe de commande commandant les mouvements du terminal (2) d'instrument à travers l'élément souple, et commandant des basculements de l'articulation en forme de serpent pour un robot chirurgical (1) à travers la structure souple de l'articulation en forme de serpent pour un robot chirurgical (1).

15. Endoscope comprenant : un système d'imagerie, une articulation en forme de serpent pour un robot chirurgical (1) selon l'une quelconque des revendications 1 à 13, un élément tubulaire et un organe de commande, dans lequel le système d'imagerie, l'articulation en forme de serpent pour un robot chirurgical (1), l'élément tubulaire et l'organe de commande sont raccordés de façon séquentielle de l'extrémité distale à l'extrémité proximale ; une extrémité proximale d'une structure souple de l'articulation en forme de serpent pour un robot chirurgical (1) étant raccordée à l'organe de commande après avoir traversé l'élément tubulaire, l'organe de commande commandant des basculements de l'articulation en forme de serpent pour un robot chirurgical (1) à travers la structure souple de l'articulation en forme de serpent pour un robot chirurgical (1), de façon à ajuster la position du système d'imagerie.

**10**

FIG. 1

**10**

FIG. 2

**10**

FIG. 3

FIG. 4

FIG. 5

11

12

FIG. 6

11

12

FIG. 7

11
12

FIG. 8

11
12

FIG. 11

11

12

FIG. 9

11

12

FIG. 10

12d

10d

11d

12c

10c

11c

12b

10b

11b

12a

10a

11a

FIG. 12

12d
11d
12c
11c
12b
11b
12a
11a

FIG. 13

12d
11d
12c
11c
12b
11b
12a
11a

FIG. 14

2

1

FIG. 15

2

10

1

20

FIG. 16

FIG. 17

FIG. 18

29

**REFERENCES CITED IN THE DESCRIPTION**

**Patent documents cited in the description**

- CN 204192566 U
- US 9387585 B2
- US 20160066937 A1
- CN 105078398 A